# EUROPEAN PATENT APPLICATION

(11) **EP 2 465 928 A1**
(43) Date of publication of application: **20.06.2012**
(21) Application number: 10195435.2
(22) Date of filing: 16.12.2010
(51) Int. Cl.: C12N 9/64, C07K 14/47, C12N 15/113, A61P 37/06, A61P 17/06, A61K 38/48, A61K 39/395, A61K 31/7088, A61K 38/07, A61K 31/513

(54) **Treatment of Th17-mediated diseases**

(71) Applicant: Academisch Medisch Centrum bij de Universiteit van Amsterdam, 1105 AZ Amsterdam (NL)
(72) Inventor: Geijtenbeek, Teunis Bernard Herman, 1076 DP Amsterdam (NL); Bouwman-Gringhuis, Sonja Inge, 3448 DX Woerden (NL)
(74) Representative: Swinkels, Bart Willem

(57) **Abstract**

A Malt1 inhibitor for preventing, delaying and/or treating a Th17-mediated disease.

## Description

### Field of the invention

A Malt1 inhibitor for preventing, delaying and/or treating a Th17-mediated disease.

### Background of the invention

Fungal infections are a major health threat and incidence of both superficial and invasive infections by *Candida* species are growing throughout the world due to increasing numbers of at-risk immunocompromised patients, such as transplant recipients and those infected with HIV-1/AIDS, as well as the emergence of strains that are resistant to antimycotic drugs [1]. Anti-fungal adaptive immunity requires both T helper cell type 1 (Th1) and Th17 immune responses; IL-17 secreted by Th17 cells mobilizes neutrophils required for anti-fungal responses [2,3], whereas Th1-produced IFNγ optimally activates neutrophils and subsequent phagocytosis of fungi [4]. Dendritic cells (DCs) are crucial for the induction of T helper cell differentiation [5,6]. Although the requirements for Th17 differentiation by human DCs are not completely clear, it is evident that secretion of IL-23, IL-1β and IL-6 are important for Th17 development [7,8], whereas IL-12p70 skews T helper cell differentiation towards Th1 responses [9]. Little is known about the molecular mechanisms that underlie the induction of the Th17-promoting cytokines by DCs after fungal infections.

Pattern recognition receptors (PRRs), such as Toll-like receptors (TLRs) and C-type lectins, sense pathogens through conserved pattern-associated molecular patterns (PAMPs), which induce signaling pathways to regulate gene transcription. C-type lectins are important in fungal recognition by DCs and in induction of anti-fungal Th1 and Th17 immune responses [5,10]. The cell-wall of many fungi, including *Candida* species (spp), consists of carbohydrate structures such as chitin, mannan and β-glucan that are recognized by C-type lectins like dectin-1, dectin-2, DC-SIGN and mannose receptor [5,11,12]. Triggering of β-glucan receptor dectin-1 by C. *albicans* induces both Th1 and Th17 immune responses by DCs through Syk-dependent NF-κB activation [10,13,14]. Syk induces the assembly of a scaffold consisting of the caspase recruitment domain 9 (CARD9) protein, B cell lymphoma 10 (Bc110) and mucosa-associated lymphoid-tissue lymphoma-translocation gene 1 (Malt1) [13,15]. This CARD9-Bc110-Maltl scaffold couples dectin-1 in human to the canonical NF-κB pathway by activating NF-κB subunit p65 and c-Rel [10,13], whereas dectin-1 triggering also leads to activation of the non-canonical NF-κB RelB pathway [10]. The balance between p65 and RelB activity is controlled by a distinct Raf-1-dependent pathway that thereby dictates expression of IL-12p70, IL-1β and IL-23 [10]. It is unclear how the CARD9-Bc110-Malt1 complex is involved in the activation of the different NF-κB subunits and how this affects Th17 differentiation. Although dectin-1-deficient people are more susceptible to mucocutaneous fungal infection, CARD9 deficiency in human causes a more pronounced phenotype with chronic mucoctaneous as well as invasive fungal infections [16,17]. These studies suggest that dectin-1 is not the only receptor that couples CARD9-Bc110-Malt1 to the defense against fungi. Indeed, dectin-2 interacts with C. *albicans* through mannan structures present on both yeast and hyphal forms [18,19] and a recent study shows that dectin-2 is involved in the induction of Th17 responses to C. *albicans* in mice [19,20]. Dectin-2 indirectly activates Syk through association with the FcRγ chain [12] which results in CARD9-dependent expression of IL-2, IL-10 and TNF [20]. Thus, both dectin-1 and dectin-2 are involved in Th17 development through Syk and CARD9 but the underlying mechanisms and involvement of Bc110 and Malt1 remain unclear. It is also unclear whether dectin-1 and dectin-2 are required for a general anti-fungal response to all *Candida* species.

Th17-mediated diseases such as inflammatory bowel diseases (Crohn's disease en Ulcerosa colitis), skin diseases (psoriasis, eczema) are treated with immunosuppressives, steroids or biologicals. 5-amino salicylic acid drugs are the standard treatment for induction and maintenance of remission in mild to moderate UC, whereas the main immunomodulators in use in inflammatory bowel disease patients are thiopurines (azathioprine, 6-mercaptopurine) and methotrexate. Anti-TNF is used as a biological in treatment of Crohn's disease. Psoriasis is a lifelong disease that requires long-term treatment and topical therapies play an important role in the treatment of psoriasis. Patients usually start with monotherapy; however, in more severe cases (> 10% body surface area, severely impaired quality of life, or recalcitrant psoriatic lesions), multiple treatment modalities may be used as part of combination, sequential, or rotational therapeutic regimens. Main treatment options include topical steroids, systemic therapies, topical vitamin D treatments such as vitamin D3 ointment, retinoids, phototherapy, and biologic therapies. Other topical therapies include the following steroid-sparing agents: coal tar, anthralin, calcineurin inhibitors, keratolytics, and emollients. For acute eczematous skin lesions, anti-inflammatory treatment consists mainly of topical glucocorticoids and topical calcineurin inhibitors (tacrolimus and pimecrolimus). Microbial colonization and superinfection may induce skin exacerbation, which can be treated by either topical or systemic antimicrobial treatment. Systemic anti-inflammatory therapy is limited to severe cases and consists of systemic steroids, cyclosporine A or mycophenolate mofetil. Novel anti-inflammatory concepts that go beyond corticosteroids are in the early phases of development. Thus, most treatments suppress the symptoms such as immune activation and inflammation but do not specifically suppress Th17 responses that cause these symptoms. This means that most therapies have side effects due to systemic immune suppression or are less effective and need to be given for long periods as maintenance of the disease. Reprogramming of the adaptive immune response by targeting induction of Th17 cells might be greatly beneficial to the patients and cause less side effects.

### Description

Here we demonstrate that dectin-1 and dectin-2 convergently contribute to anti-fungal Th17 immunity by inducing IL-1β and IL-23 production. Both dectin-1 and dectin-2 triggering leads to Malt1 activation, which specifically activates NF-κB subunit c-Rel that is pivotal to the transcriptional activation of the *Il1b and I123p19* genes. Syk-dependent recruitment of CARD9 and Bc110 upon dectin-1 triggering is crucial for activation of all NF-κB subunits, while recruitment of Malt1 and activation of its paracaspase activity is distinctively required for c-Rel but not p65 or RelB activation. In contrast to dectin-1, dectin-2 triggering activates only c-Rel, which is also dependent on Malt1 signaling, signifying a specialized function for dectin-2 in Th17 immunity. Simultaneous triggering of dectin-1 and dectin-2 by pathogenic fungi promotes the expression of IL-1β and IL-23 to boost Th17-mediated cellular responses, whereas Malt1 inhibition after *Candida* infection markedly reduces Th17 polarization. Thus, Malt1 activation links dectin-1 and dectin-2 to the c-Rel-dependent expression of IL- 1βand IL-23 and directs adaptive anti-fungal immunity.

We believe the inhibition of a Malt1 protein as identified herein is a specific, safe and therefore non-toxic way of preventing, delaying and/or treating a Th17-mediated disease.

### Malt1 inhibitor

In a first aspect, there is provided a Malt1 inhibitor for preventing, delaying and/or treating a Th17-mediated disease.

A Th17-mediated disease is a disease which is induced or mediated by T helper type 17 (also identified as Th17 cells) cells. Such diseases may be characterized by increased presence of IL-23, T helper 17 cells, IL-17 and/or IL-22 compared to the average amount of IL-23, IL-17 and/or IL22 and/or T helper 17 cells present in healthy donors. In this context, Th17 cells are preferably activated. It means that these cells will proliferate and subsequently express IL-17. Dendritic cells have the ability to induce the differentiation of CD4 T cells into T helper type 17 cells. This ability may be assessed in the polarization assay described in the example. T helper type 17 cells are characterized by their ability to produce IL-17. The presence of IL-17, IL-23, IL-22 may be assessed by ELISA or by flow cytometry by intracellular staining for IL-17 as described in the examples.

Preferably, an increase or an up-regulation of IL-17, IL-23 and/or IL-22 means an increase of at least 5% of the expression level of a nucleotide sequence using real-time PCR, arrays or Northern blot. More preferably, an increase of the expression level of a nucleotide sequence means an increase of at least 10%, even more preferably at least 20%, at least 30%, at least 40%, at least 50%, at least 70%, at least 90%, at least 100%, or more. In another preferred embodiment, an increase of the expression level of IL-17, IL-23 and/or IL-22 means an increase of at least 5% of the expression level of IL-17, IL-23 and/or IL-22 using western blotting and/or using ELISA or a suitable assay. More preferably, an increase of the expression level of IL-17, IL-23 and/or IL-22 means an increase of at least 10%, even more preferably at least 20%, at least 30%, at least 40%, at least 50%, at least 70%, at least 90%, at least 150% or more.

Preferably, An increase of Th17 helper, preferably activated Th17 helper cells means an increase of at least 5% of the number of these cells assessed using intracellular FACS staining as described in the examples for assessing the presence of IL-17. More preferably, an increase of activated Th17 helper cells means an increase of at least 10%, even more preferably at least 20%, at least 30%, at least 40%, at least 50%, at least 70%, at least 90%, at least 100%, or more.

In a preferred embodiment, this increase ofIL-17, IL23 and/or IL-22 and/or Th 17 helper cells, preferably activated Th17 helper cells is an increase which is present during at least one week, one month, six month, one year or more. This increase may even be constitutive if in said individual for more than one or more than two years said increase is present. This increase is assessed by comparison to the corresponding average level of IL-17, IL-23, IL-22 and/or Th17 helper cells, preferably activated Th17 helper cells in a healthy person.

Throughout the invention, an individual or a subject or a patient is preferably a human being.

Within the context of the invention, a Th17-mediated disease is preferably a Th 17-mediated autoimmune disease and/or skin disease.

A Th17-mediated autoimmune disease may be any auto-immune disease for which Th17 cells are known to play a role. A preferred auto-immune disease in this context is the Crohn's disease also called inflammatory bowel disease, multiple sclerosis, and rheumatoid arthritis.

A Th17-mediated disease may also be a skin disease. The invention is not limited to a specific skin disease. Any skin disease known to be induced or mediated by a fungus or a bacterium is encompassed within the scope of the present invention. Preferred skin diseases in this context are Psoriasis, eczema or as a skin disease caused by a fungus and/or bacterium, atopic dermatitis. Eczema may be allergic eczema.

A Malt1 inhibitor is a compound which is able to decrease an activity of Malt1 and/or to decrease its expression level and/or sub cellular localisation.

Throughout the application, Malt1 is a protein being represented by an amino acid sequence, said amino acid sequence being encoded by a nucleotide sequence selected from:
(a) a nucleotide sequence that has at least 60, 70, 80, 85, 90, 95, 98 or 99% identity with nucleotide sequence SEQ ID: 1 or 2; and,
(b) a nucleotide sequence that encodes an amino acid sequence that has at least 60, 70, 80, 85, 90, 95, 98 or 99% amino acid identity with an amino acid sequence encoded by a nucleotide sequence selected from SEQ ID NO: 1 or 2.

A preferred nucleotide sequence encoding Malt1 is represented by SEQ ID NO: 1 or 2.. A corresponding preferred amino acid sequence of Malt1 is represented by SEQ ID NO:3 or 4.

A "decrease of an activity of Malt1 or a decrease of the expression level of Malt1 is herein understood to mean any detectable change in a biological activity exerted by Malt1 or in the expression level of Malt1 as compared to said activity or expression of a wild type Malt1 such as the one encoded by SEQ ID NO:1 or 2. The decrease of the level or of the amount of a nucleotide encoding Malt1 is preferably assessed using classical molecular biology techniques such as (real time) PCR, arrays or Northern analysis. Alternatively, according to another preferred embodiment, the decrease of the expression level of Malt1 is determined directly by quantifying the amount of a Malt1 protein. Quantifying a protein amount may be carried out by any known technique such as Western blotting or immunoassay using an antibody raised against said protein. The skilled person will understand that alternatively or in combination with the quantification of a nucleic acid sequence and/or the corresponding polypeptide, a quantification of a substrate or a quantification of the expression of a target gene of Malt1 or of any compound known to be associated with a function or activity of Malt1 or the quantification of said function or activity of Malt1 using a specific assay may be used to assess the decrease of an activity or expression level of Malt 1. Several assays are suitable to measure a Malt1 activity.

In this context, a preferred activity of a Malt1 is a proteolytic activity, more preferably a paracaspase activity. In this context, a preferred activity of Malt1 is the induction of the activation of the c-Rel subunit of NF-κB and/or the induction of the production of IL-1b and/or the induction of the production of IL-23p19. A preferred assay for assessing the activation of the c-Rel subunit of NF-κB is to assess its nucleus translocation using immunofluorescence staining on cells, preferably as described in the example. Another assay for assessing the activation of the c-Rel subunit of NF-κB is to assess its DNA binding ability by a chromatin immunoprecipitation assay, preferably as described in the example. A preferred assay for assessing the production of IL-1b and/or the induction of the production of IL-23p19 is ELISA on supernatants of cells, preferably as described in the example.

Preferably, a decrease or a down-regulation of the expression level of a nucleotide sequence encoding a Malt1 protein means a decrease of at least 5% of the expression level of a nucleotide sequence using real-time PCR, arrays or Northern blot. More preferably, a decrease of the expression level of a nucleotide sequence means a decrease of at least 10%, even more preferably at least 20%, at least 30%, at least 40%, at least 50%, at least 70%, at least 90%, at least 100%, or more. Preferably, the expression is no longer detectable. In another preferred embodiment, a decrease of the expression level of a Malt1 protein means a decrease of at least 5% of the expression level of a protein using western blotting and/or using ELISA or a suitable assay. More preferably, a decrease of the expression level of a Malt1 protein means a decrease of at least 10%, even more preferably at least 20%, at least 30%, at least 40%, at least 50%, at least 70%, at least 90%, at least 150% or more. Preferably, the expression is no longer detectable.

In another preferred embodiment, a decrease, an inhibition or a reduction of a Malt1 activity means a decrease, an inhibition or a reduction of at least 5% of a Malt1 activity as defined herein using a suitable assay as earlier defined herein. More preferably, a decrease, an inhibition or a reduction of a Malt1 activity means a decrease, an inhibition, or a reduction of at least 10%, even more preferably at least 20%, at least 30%, at least 40%, at least 50%, at least 70%, at least 90%, at least 150% or more. Preferably, said activity is no longer detectable.

A preferred Malt1 inhibitor is able to reduce, inhibit or decrease the activation of the subunit c-Rel of NF-κB and/or reduce, inhibit or decrease the production of IL-1b and/or of IL-23p19. This is preferably assessed using one of the assays as earlier described herein.

An inhibitor of Malt1 or a Malt1 inhibitor may be any compound. The invention also provides a method for identifying additional inhibitors of Malt1 (see later herein). Preferably a Malt1 inhibitor is a DNA or RNA molecule, a dominant negative molecule of Malt1, an inhibiting antibody raised against Malt1, a small molecule such as a peptide-like molecule (referred to as peptidomimetics) or a non-peptide molecule. Each of these inhibitors is presented in more details below.

An inhibitor may act at the level of the protein itself, e.g. by providing an antagonist or inhibitor of a Malt1 protein to a cell, such as e.g. an inhibiting antibody raised against Malt1 (named an antibody herein) or a dominant negative form of Malt1 or an antisense for Malt1 (named antisense molecule herein). An antibody, an antisense molecule or a dominant negative form of a Malt1 of the invention may be obtained as described below. Alternatively, an inhibitor may act at the level of the nucleotide encoding Malt1. In this case, the expression level of Malt1 is decreased by regulating the expression level of a nucleotide sequence encoding Malt1.

An antisense molecule is a molecule capable of inhibiting the biosynthesis (usually the translation) of a nucleotide sequence encoding Malt1. Decreasing gene expression by providing antisense or interfering RNA molecules is described below herein and is e.g. reviewed by Famulok et al. (2002, Trends Biotechnol., 20(11): 462-466). An antisense molecule may be provided to a cell as such or it may be provided by introducing an expression construct into a cell, whereby said expression construct comprises an antisense nucleotide sequence that is capable of inhibiting the expression of a nucleotide sequence encoding a Malt1 protein, and whereby said antisense nucleotide sequence is under control of a promoter capable of driving transcription of said antisense nucleotide sequence in a cell. The expression level of a Malt1 protein may also be down-regulated or decreased by introducing an expression construct into a cell, whereby said expression construct comprises a nucleotide sequence encoding a factor capable of trans-repression of an endogenous nucleotide sequence encoding a Malt1 protein. Preferably, a nucleotide sequence capable of trans-repression of an endogenous nucleotide sequence is a dominant negative of said endogenous nucleotide sequence as exemplified below.

An antisense or interfering nucleic acid molecule may be introduced into a cell directly "as such", optionally in a suitable formulation, or it may be produced *in situ* in a cell by introducing into a cell an expression construct or nucleic acid construct comprising a (antisense or interfering) nucleotide sequence that is capable of inhibiting the expression of a nucleotide sequence encoding Malt1, whereby, optionally, an antisense or interfering nucleotide sequence is under control of a promoter capable of driving expression of said nucleotide sequence in a cell. Preferably, the cell is an antigen presenting cell (APC), either macrophage, dendritic cell or Langerhans cell. Such a nucleic acid construct of the invention comprises or consists of a nucleotide sequence that encodes an RNAi agent, i.e. an RNA molecule that is capable of RNA interference or that is part of an RNA molecule that is capable of RNA interference. Such an RNA molecule is referred to as siRNA (short interfering RNA, including e.g. a short hairpin RNA). A nucleotide sequence that encodes an RNAi agent preferably has sufficient complementarity with a cellular nucleotide sequence encoding Malt1 as earlier defined herein to be capable of inhibiting or decreasing the expression of said protein Malt1. Preferred siRNAs comprise or consist of SEQ ID NO: 5-8.

Alternatively or in combination with the antisense approach, one may also use an inactivating approach. In this approach, an inactivating nucleic acid construct is introduced into a cell. Said inactivating construct comprises or consists of a nucleotide molecule which is designed in order to inactivate the expression of the Malt1 protein. The skilled person knows how to design an inactivating construct (Capecchi 1989, Science Jun 16;244(4910):1288-92). For example, at least part of a Malt1 gene is replaced by a marker such as the neomycine gene.

Alternatively or in combination with the antisense and inactivating approaches, one may also use a dominant negative approach. In this approach, a nucleic acid construct is introduced into a cell, wherein said nucleic construct comprises a dominant negative nucleotide sequence that is capable of inhibiting or down-regulating an activity of a corresponding endogenous Malt1 protein, and wherein, optionally, a dominant negative nucleotide sequence is under the control of a promoter capable of driving expression of said dominant negative nucleotide sequence in a cell.

In a nucleic acid construct of the invention (dominant negative approach or antisense approach), a promoter or a regulatory region preferably is a promoter or regulatory region that is specific for An APC cell. A promoter that is specific for an APC cell is a promoter with a transcription rate that is higher in said cell than in other types of cells. Preferably the promoter's transcription rate in said cell is at least 1.1, 1.5, 2.0 or 5.0 times higher than in other cell. A promoter for use in a DNA construct of the invention is preferably of mammalian origin, more preferably of human origin. For expression in an APC cell, a preferred promoter is a promoter of a DC-SIGN or of a Langerin gene. In a preferred embodiment, a nucleic acid construct is a viral gene therapy vector selected from gene therapy vectors based on an adenovirus, an adeno-associated virus (AAV), a herpes virus, a pox virus and a retrovirus. A preferred viral gene therapy vector is an AAV or Lentiviral vector. Such vectors are further described herein below. Several Malt1 inhibitors are commercially available and suitable to be used in the present invention. The first one is a paracaspase inhibitor called z-VRPR-FMK(Malt1 inhibitor [22]; Alexis, ALX-260-166): z-Valine-Arginine-Proline-DL-Arginine-FMK with a formula C₃₁H₄₉FN₁₀0₆-C₂HF₃0₂. Other Malt1 inhbitors are extensively disclosed in US20040110145 and WO 2009/065897. Preferred Malt1 inhibitors identified in WO 2009/065897 comprise a protein, such as a serpin, a peptide or a peptide derivate such as Z-LSSR-CHO, Z-LSSR-CMK, Z-GASR-CHO or Z-GASR-CMK, or a small chemical compound, such as 5-{[5-(3-chloro-4-methylphenyl)-2-furyl]methylene}-2-thioxodihydro-4,6(1 H,5H)-pyrimidinedione.

Accordingly in a preferred embodiment, a Malt1 inhibitor is a DNA/RNA molecule, an antibody, a peptide or a peptide derivate as Z-VRPR-FMK, Z-LSSR-CHO, Z-LSSR-CMK, Z-GASR-CHO or Z-GASR-CMK, or a chemical compound, such as 5-{[5-(3-chloro-4-methylphenyl)-2- furyl]methylene}-2-thioxodihydro-4,6(1 H,5H)-pyrimidinedione.

### Composition

In a further aspect, there is provided a composition comprising a Malt1 as defined herein for preventing, delaying and/or treating a Th17-mediated disease. A preferred composition or preparation is a pharmaceutical composition or preparation comprising as active ingredient an inhibitor of Malt1 a, preferably at least one of an antibody, a dominant negative, a nucleic acid, an antisense molecule, a nucleic acid construct or a gene therapy vector as defined above or any other known Malt1. The composition preferably at least comprises a pharmaceutically acceptable carrier in addition to an active ingredient.

In some embodiments, a nucleic acid or nucleic acid construct or antibody or dominant negative of the invention as purified from mammalian, insect or microbial cell cultures, from milk of transgenic mammals or other source is administered in purified form together with a pharmaceutical carrier as a pharmaceutical composition. A method of producing a pharmaceutical composition comprising a polypeptide is described in US Patents No.'s 5,789,543 and 6,207,718. The preferred form depends on the intended mode of administration and therapeutic application.

A pharmaceutical carrier can be any compatible, non-toxic substance suitable to deliver an antibody, a dominant negative molecule or a nucleic acid or a gene therapy vector to the patient or any other Malt1 inhibitor. Sterile water, alcohol, fats, waxes, and inert solids may be used as the carrier. A pharmaceutically acceptable adjuvant, buffering agent, dispersing agent, and the like, may also be incorporated into a pharmaceutical composition.

The concentration of an antibody or dominant negative or nucleic acid of nucleic acid construct of the invention or any other Malt1 inhibitor in a pharmaceutical composition can vary widely, i.e., from less than about 0.1 % by weight, usually being at least about 1% by weight to as much as 20% by weight or more.

For oral administration, an active ingredient can be administered in solid dosage forms, such as capsules, tablets, and powders, or in liquid dosage forms, such as elixirs, syrups, and suspensions. Active component(s) can be encapsulated in gelatin capsules together with inactive ingredients and powdered carriers, such as glucose, lactose, sucrose, mannitol, starch, cellulose or cellulose derivatives, magnesium stearate, stearic acid, sodium saccharin, talcum, magnesium carbonate and the like. An example of an additional inactive ingredient that may be added to provide desirable colour, taste, stability, buffering capacity, dispersion or other known desirable features are red iron oxide, silica gel, sodium lauryl sulfate, titanium dioxide, edible white ink and the like. A similar diluent can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric-coated for selective disintegration in the gastrointestinal tract. Liquid dosage forms for oral administration can contain coloring and flavoring to increase patient acceptance.

An antibody or dominant negative molecule or nucleic acid or nucleic acid construct or gene therapy vector is preferably administered parentally. An antibody or dominant negative molecule or nucleic acid or nucleic acid construct or vector for a preparation for parental administration must be sterile. Sterilization is readily accomplished by filtration through sterile filtration membranes, prior to or following lyophilisation and reconstitution. The parental route for administration of an antibody or dominant negative molecule or nucleic acid or nucleic acid construct or vector is in accord with known methods, e.g. injection or infusion by intravenous, intraperitoneal, intramuscular, intraarterial, intralesional, intracranial, intrathecal, transdermal, nasal, buccal, rectal, or vaginal routes. An antibody or dominant negative molecule or nucleic acid or nucleic acid construct or vector is administered continuously by infusion or by bolus injection. A typical composition for intravenous infusion could be made up to contain 10 to 50 ml of sterile 0.9% NaCl or 5% glucose optionally supplemented with a 20% albumin solution and 1 to 50 µg of a polypeptide, antibody or dominant negatif or nucleic acid or nucleic acid construct or vector. A typical pharmaceutical composition for intramuscular injection would be made up to contain, for example, 1 - 10 ml of sterile buffered water and 1 to 100 µg of an antibody or dominant negative molecule or nucleic acid or nucleic acid construct or vector of the invention. Methods for preparing a parenterally administrable composition is well known in the art and described in more detail in various sources, including, for example, Remington's Pharmaceutical Science (15th ed., Mack Publishing, Easton, PA, 1980) (incorporated by reference in its entirety for all purposes).

A composition comprising a Malt1 inhibitor may comprise an excipient that will aid in delivery of each of the constituents as defined herein to a cell and/or into a cell, preferably an APC cell as a dendritic cell, langerhans cell, or a macrophage. Preferred are excipients capable of forming complexes, nanoparticles, micelles, vesicles, antibody, sugars and/or liposomes that deliver each constituent as defined herein, complexed or trapped in a vesicle or liposome through a cell membrane. An antibody raised against a marker specifically expressed by an APC cell could be used to this end. Examples of such markers include DC-SIGN, Langerin and the Mannose receptor. Alternatively a sugar may be coupled or associated with or linked to a vesicle such as a liposome. A sugar may be a mannose and/or a fucose and/or a carbohydrate containing mannose or fucsoe structure(s). Many of these excipients are known in the art. Suitable excipients comprise polyethylenimine (PEI), or similar cationic polymers, including polypropyleneimine or polyethylenimine copolymers (PECs) and derivatives, synthetic amphiphils (SAINT-18), lipofectinTM, DOTAP and/or viral capsid proteins that are capable of self assembly into particles that can deliver each constitutent as defined herein to a cell, preferably an APC cell. Such excipients have been shown to efficiently deliver nucleic acids for example to a wide variety of cultured cells. Their high transfection potential is combined with an excepted low to moderate toxicity in terms of overall cell survival. The ease of structural modification can be used to allow further modifications and the analysis of their further *(in vivo)* nucleic acid transfer characteristics and toxicity.

Lipofectin represents an example of a liposomal transfection agent. It consists of two lipid components, a cationic lipid N-[1-(2,3 dioleoyloxy)propyl]-N,N,N-trimethylammonium chloride (DOTMA) (cp. DOTAP which is the methylsulfate salt) and a neutral lipid dioleoylphosphatidylethanolamine (DOPE). The neutral component mediates the intracellular release. Another group of delivery systems are polymeric nanoparticles.

Polycations such like diethylaminoethylaminoethyl (DEAE)-dextran, which are well known as DNA transfection reagent can be combined with butylcyanoacrylate (PBCA) and hexylcyanoacrylate (PHCA) to formulate cationic nanoparticles that can deliver each constituent as defined herein, preferably a nucleic acid across cell membranes into cells.

In addition to these common nanoparticle materials, the cationic peptide protamine offers an alternative approach to formulate an inhibitor with colloids. This colloidal nanoparticle system can form so called proticles, which can be prepared by a simple self-assembly process to package and mediate intracellular release of an inhibitor. The skilled person may select and adapt any of the above or other commercially available alternative excipients and delivery systems to package and deliver an inhibitor for use in the current invention to deliver it for the prevention or treatment of a Th17-mediated disease in humans.

In preferred embodiment, an inhibitor is formulated in a so-called slow release formulation or slow release vehicle. Such formulations are also named formulation with a delayed or controlled release. A controlled release formulation is a formulation that will release at least 20%, at least 25%, at least 30%, at least 35%, at least 40%, at least 45%, at least 50%, at least 55%, at least 60%, at least 65%, at least 70%, at least 75%, at least 80% of its active ingredient in a controlled fashion, i.e. a Malt1 inhibitor within a day, a week, two weeks, three weeks, a month, or longer. Several types of slow release formulations are already known such as mineral oil (e.g. Montanide ISA 51) or Poly-lactic-co-glycolic acid (PLGA) or polymer based formulations. An example of a polymer-based formulation is a gel composition comprising charged polymers as described in WO 2005/110377 or a composition comprising a dextran hydrogel as described in WO 02/17884 or WO 2005/051414 or US 3,710,795.

For therapeutic applications, a pharmaceutical composition is administered to a patient suffering from a Th17-mediated disease in an amount sufficient to reduce the severity of a symptom and/or prevent or arrest further development of said symptom. An amount adequate to accomplish this is defined as a "therapeutically-" or "prophylactically-effective dose". Such effective dosages will depend on the severity of the condition and on the general state of the patient's health. In general, a therapeutically- or prophylactically-effective dose preferably is a dose, which is sufficient to reverse the symptoms to the average levels found in normal unaffected healthy individuals.

A Malt1 inhibitor as defined herein is preferably able to reduce the severity of a symptom if after at least one week, one month, six month, one year or more of treatment, the severity of said symptom is said to have been reduced. Reduction or alleviation in this context may mean that said symptom had been significantly changed towards the absence of said symptom which is characteristic for a healthy person and/or towards a change of said symptom that corresponds to the state of the same individual at the onset of the treatment. Said reduction of the severity of a symptom is preferably assessed using a dose of a Malt1 inhibitor as identified herein.

A preferred symptom associated with a Th17-mediated disease is inflammation and increased neutrophil influx at site of inflammation.

Alternatively or in combination with the effect of a pharmaceutical composition on a symptom as identified above, for therapeutic applications, a pharmaceutical composition is administered to a patient suffering from a Th17-mediated disease in an amount sufficient to alter the value of a parameter known to be associated with said disease towards a value of a healthy person. An amount adequate to accomplish this is defined as a "therapeutically-" or "prophylactically-effective dose". Such effective dosages will depend on the severity of the condition and on the general state of the patient's health. In general, a therapeutically- or prophylactically-effective dose preferably is a dose, which is sufficient to alter a parameter known to be associated with said disease to the average value found in normal unaffected healthy individuals.

A Malt1 inhibitor as defined herein is preferably able to alter the value of a parameter if after at least one week, one month, six month, one year or more of treatment, the value of said parameter is said to have been altered. Alteration in this context may mean that said parameter had been significantly changed towards a value of said parameter which is characteristic for a healthy person and/or towards a value of said parameter that corresponds to the state of the same individual at the onset of the treatment. In a preferred embodiment, the value of a parameter had been improved. Improvement in this context may mean that said parameter had been significantly changed towards a value of said parameter for a healthy person and/or towards a value of said parameter that corresponds to the value of said parameter in the same individual at the onset of the treatment. Said alteration of the value of a parameter is preferably assessed using a dose of a Malt1 inhibitor as identified herein.

A preferred parameter associated with a Th17-mediated disease is the amount ofIL-17 and/ or IL-22 produced, the activation level of c-Rel, the amount of IL-1b and/orIL-23 produced. Assays for assessing any of these parameters have already been defined herein.

Accordingly in a preferred embodiment, a parameter is IL-17 and a change is a decrease of the amount of IL-17 as identified herein. Accordingly in another preferred embodiment, a parameter is C-rel and a change is a decrease or reduction of its activation as identified herein.

Accordingly in another preferred embodiment, a parameter is IL-1b and/or IL-23p19 and a change is a decrease or reduction of the production of IL-1b and/or IL-23p19 as identified herein.

A Malt1 inhibitor according to the invention is suitable for direct administration to a cell, tissue and/or organ *in vivo* of an individual affected by or at risk of developing a Th17-mediated disease and may be administered directly *in vivo, ex vivo* or *in vitro.* An individual or a subject or a patient is preferably a mammal, more preferably a human being.

In a preferred embodiment, a concentration of a Malt1 inhibitor is ranged from 0.01 nM to 1 µM is used. More preferably, the concentration used is from 0.05 to 400 nM, or from 0.1 to 400 nM, or from 0.02 to 400 nM, or from 0.05 to 400 nM, even more preferably from 1 to 200 nM. Preferred concentrations are from 0.1 nM to 1 µM. More preferably, the concentration used is from 0.3 to 400 nM, even more preferably from 1 to 200 nM. Dose ranges of a Malt1 inhibitor are preferably designed on the basis of rising dose studies in clinical trials *(in vivo* use) for which rigorous protocol requirements exist. In a present method of the invention, an antibody or dominant negative molecule or nucleic acid or nucleic acid construct or vector is usually administered at a dosage of about 1 µg/kg patient body weight or more per week to a patient. Often dosages are greater than 10 µg/kg per week. A dosage regime can range from 10 µg/kg per week to at least 1 mg/kg per week. Typically a dosage regime may be 10 µg/kg per week, 20 µg/kg per week, 30 µg/kg per week, 40 µg/kg week, 60 µg/kg week, 80 µg/kg per week and 120 µg/kg per week. In a preferred regime 10 µg/kg, 20 µg/kg or 40 µg/kg is administered once, twice or three times weekly. Treatment is preferably administered by parenteral route.

The ranges of concentration or dose of a Malt1 inhibitor are preferred concentrations or doses *for in vitro* or *ex vivo* uses. The skilled person will understand that depending on the identity of the Malt1 inhibitor used, the target cell to be treated, the gene target and its expression levels, the medium used and the transfection and incubation conditions, the concentration or dose of compound used may further vary and may need to be optimised any further.

In addition, an inhibitor could be covalently or non-covalently linked to a targeting ligand specifically designed to facilitate the uptake in to a cell, cytoplasm and/or its nucleus. Such ligand could comprise (i) a molecule (including but not limited to peptide (-like) structures) recognising cell, tissue or organ specific elements facilitating cellular uptake and/or (ii) a molecule able to facilitate the uptake in to a cell and/or the intracellular release of an inhibitor from vesicles, e.g. endosomes or lysosomes.

Therefore, in a preferred embodiment, an inhibitor is formulated in a composition or a medicament which is provided with at least an excipient and/or a targeting ligand for delivery and/or a delivery device thereof to a cell and/or enhancing its intracellular delivery. Accordingly, the invention also encompasses a pharmaceutically acceptable composition comprising an inhibitor and further comprising at least one excipient and/or a targeting ligand for delivery and/or a delivery device of said inhibitor to a cell and/or enhancing its intracellular delivery.

Depending on their identity, the skilled person will know which type of formulation is the most appropriate for each constituent as defined herein.

In a preferred embodiment, a targeting part is added to a Malt1 inhibitor to ensure said inhibitor is targeted to an APC cell, preferably a dendritic cell. This may lower possible side effects and/or may improve the specificity of said inhibitor. A targeting part may be an antibody that recognizes a protein expressed by the cell it needs to be targeted to, or a ligand specific for a receptor that is expressed by the cell it needs to be targeted to, or derivatives thereof. Specific targeting part or excipients have been identified earlier herein as including nanoparticles, micelles, vesicles, antibody, sugars and/or liposomes. An antibody raised against a marker specifically expressed by an APC cell could be used to this end. Examples of such markers include DC-SIGN, Langerin and the Mannose receptor. Alternatively a sugar may be coupled or associated with or linked to a vesicle such as a liposome. A sugar may be mannose and/or fucose. A targeting part may be used in this context when the targeting of an inhibitor linked to this targeting part to APC is increased of at least 5%, 10%, 15%, 20%, 25%, 30%, 35%, 40%, 45%, 50%, 55%, 60%, 65%, 70%, 75%, 80%, 85%, 90%, 95%, 100% or more compared to the targeting of corresponding inhibitor without targeting part. The targeting to APC may be assessed using in vivo imaging techniques. To detect whether a Malt1 has been targeted, said inhibitor may be radio labeled or fluorescently labeled. A targeted compound may be conjugated to a substance that allows radioactive and fluorescent imaging, positron emission tomography (PET) scanning, magnetic resonance imaging (MRI) scanning, or X-ray/ computed tomography (CT) scanning. Examples of radioactive substances are radioactive labels such as Indium-11, Technetium-99m, Iodine-131 or Fluorine-19. Examples of fluorescent substances are the fluorescent dye fluorescein isothiocyanate or aliphatic biodegradable photoluminescent polymers. The fluorescent or photoluminescent probes may also be present in a formulation that comprises a Malt1 inhibitor, such as a liposomal formulation or a nanoparticle.

### Use

In a further aspect, there is provided the use of a Malt1 inhibitor or a composition as defined herein for the manufacture of a medicament for preventing, delaying and/or treating a Th17-mediated disease. All features have already been defined herein.

### Method

In a further aspect, there is provided a method for preventing, delaying and/or treating a Th17-mediated disease wherein use is made of a Malt1 inhibitor or a composition as defined herein. All features have already been defined herein.

A method of the invention preferably comprises the step of administering to a subject a therapeutically effective amount of a pharmaceutical composition comprising an inhibitor of Malt1, preferably an inhibiting antibody, a DNA or an RNA, a dominant negative molecule or a nucleic acid construct for decreasing an activity or expression level of a Malt1 as defined herein. A nucleic acid construct is for inhibiting expression of a Malt1 protein of the invention such as an antisense molecule or an RNA molecule capable of RNA interference as defined herein. Alternatively or in combination with both previous embodiments, a nucleic acid construct comprising a dominant negative of an endogenous Malt1 protein may be administered into a cell or into a subject. In a method of the invention, a pharmaceutical composition comprising a nucleic acid construct is preferably administered in a cell or in a subject. Preferably, the subject is a mammal. More preferably the mammal is a human being. Each inhibitor may be first applied or administrated in vitro or ex vivo in a cell. More preferably a cell is a dendritic cell.

In a further preferred method, another treatment may be applied in combination with a Malt1 inhibitor. This combined use may be a sequential use: each component is administered in a distinct composition. Alternatively each compound may be used together in a single composition. Another treatment in this context may be an immunosuppressive treatment as one including an immunosuppressive agent as a steroid.

### Screening method

In a further aspect, there is provided a method for identification of a compound inhibiting Malt1, the method comprising the steps of:
a) providing a test cell population capable of expressing a Malt1;
b) contacting the test cell population with the compound;
c) determining an activity of Malt1 in the test cell population contacted with the compound;
d) comparing the activity level determined in (c) with the corresponding activity in a test cell population that is not contacted with the compound; and,
e) identifying a compound that produces a difference in activity of Malt1, between the test cell population that is contacted with the compound and the test cell population that is not contacted with the compound.

This method is a so-called cell-based method. Preferably, in a method a test cell population comprises eukaryotic cells. A test cell population preferably comprises mammalian cells, more preferably human cells. More preferred human cells are dendritic cells and/or other cells or cell lines described in the Examples herein. Raji cells may be used in this context.

In one aspect, the invention also pertains to a substance that has been identified in one of said methods. A decrease in expression level or of an activity has preferably the same meaning as given earlier herein.

### General technical information

### Sequence identity

"Sequence identity" is herein defined as a relationship between two or more amino acid (polypeptide or protein) sequences or two or more nucleic acid (polynucleotide) sequences, as determined by comparing the sequences. The identity between two nucleic acid sequences is preferably defined by assessing their identity within a whole SEQ ID NO as identified herein or part thereof. Part thereof may mean at least 50% of the length of the SEQ ID NO, or at least 60%, or at least 70%, or at least 80%, or at least 90%.

In the art, "identity" also means the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods, including but not limited to those described in (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48:1073 (1988).

Preferred methods to determine identity are designed to give the largest match between the sequences tested. Methods to determine identity and similarity are codified in publicly available computer programs. Preferred computer program methods to determine identity and similarity between two sequences include e.g. the GCG program package (Devereux, J., et al., Nucleic Acids Research 12 (1): 387 (1984)), BestFit, BLASTP, BLASTN, and FASTA (Altschul, S. F. et al., J. Mol. Biol. 215:403-410 (1990). The BLAST X program is publicly available from NCBI and other sources (BLAST Manual, Altschul, S., et al., NCBI NLM NIH Bethesda, MD 20894; Altschul, S., et al., J. Mol. Biol. 215:403-410 (1990). The well-known Smith Waterman algorithm may also be used to determine identity.

Preferred parameters for polypeptide sequence comparison include the following: Algorithm: Needleman and Wunsch, J. Mol. Biol. 48:443-453 (1970); Comparison matrix: BLOSSUM62 from Hentikoff and Hentikoff, Proc. Natl. Acad. Sci. USA. 89:10915-10919 (1992); Gap Penalty: 12; and Gap Length Penalty: 4. A program useful with these parameters is publicly available as the "Ogap" program from Genetics Computer Group, located in Madison, WI. The aforementioned parameters are the default parameters for amino acid comparisons (along with no penalty for end gaps). Preferred parameters for nucleic acid comparison include the following: Algorithm: Needleman and Wunsch, J. Mol. Biol. 48:443-453 (1970); Comparison matrix: matches=+10, mismatch=0; Gap Penalty: 50; Gap Length Penalty: 3. Available as the Gap program from Genetics Computer Group, located in Madison, Wis. Given above are the default parameters for nucleic acid comparisons.

Optionally, in determining the degree of amino acid similarity, the skilled person may also take into account so-called "conservative" amino acid substitutions, as will be clear to the skilled person. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to ser; Arg to lys; Asn to gln or his; Asp to glu; Cys to ser or ala; Gln to asn; Glu to asp; Gly to pro; His to asn or gln; Ile to leu or val; Leu to ile or val; Lys to arg; gln or glu; Met to leu or ile; Phe to met, leu or tyr; Ser to thr; Thr to ser; Trp to tyr; Tyr to trp or phe; and, Val to ile or leu.

### Recombinant techniques and methods for recombinant production of a Malt1 polypeptide or protein or a dominant negative Malt1 polypeptide or protein named herein a polypeptide

A polypeptide for use in the present invention can be prepared using recombinant techniques, in which a nucleotide sequence encoding a polypeptide of interest is expressed in a suitable host cell. The present invention thus also concerns the use of a vector comprising a nucleic acid molecule represented by a nucleotide sequence as defined above. Preferably a vector is a replicative vector comprising an origin of replication (or autonomously replication sequence) that ensures multiplication of a vector in a suitable host for the vector. Alternatively a vector is capable of integrating into a host cell's genome, e.g. through homologous recombination or otherwise. A particularly preferred vector is an expression vector wherein a nucleotide sequence encoding a polypeptide as defined above, is operably linked to a promoter capable of directing expression of a coding sequence in a host cell for the vector.

As used herein, the term "promoter" refers to a nucleic acid fragment that functions to control the transcription of one or more genes, located upstream with respect to the direction of transcription of the transcription initiation site of the gene, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active under most physiological and developmental conditions. An "inducible" promoter is a promoter that is regulated depending on physiological or developmental conditions. A "tissue specific" promoter is only active in specific types of differentiated cells/tissues, such as preferably intestinal cells or tissues.

An expression vector allows a polypeptide of the invention as defined above to be prepared using recombinant techniques in which a nucleotide sequence encoding a polypeptide of interest is expressed in a suitable cell, e.g. cultured cells or cells of a multicellular organism, such as described in Ausubel et al., "Current Protocols in Molecular Biology", Greene Publishing and Wiley-Interscience, New York (1987) and in Sambrook and Russell (2001, *supra);* both of which are incorporated herein by reference in their entirety. Also see, Kunkel (1985) Proc. Natl. Acad. Sci. 82:488 (describing site directed mutagenesis) and Roberts et al. (1987) Nature 328:731-734 or Wells, J.A., et al. (1985) Gene 34: 315 (describing cassette mutagenesis).

Typically, a nucleic acid encoding a polypeptide of the invention is used in an expression vector. The phrase "expression vector" generally refers to a nucleotide sequence that is capable of effecting expression of a gene in a host compatible with such sequences. These expression vectors typically include at least a suitable promoter sequence and optionally, a transcription termination signal. Additional factors necessary or helpful in effecting expression can also be used as described herein. A nucleic acid or DNA encoding a polypeptide is incorporated into a DNA construct capable of introduction into and expression in an *in vitro* cell culture. Specifically, a DNA construct is suitable for replication in a prokaryotic host, such as bacteria, *e.g., E. coli,* or can be introduced into a cultured mammalian, plant, insect, e.g., Sf9, yeast, fungi or another eukaryotic cell line.

A DNA construct prepared for introduction into a particular host typically include a replication system recognized by the host, the intended DNA segment encoding a desired polypeptide, and transcriptional and translational initiation and termination regulatory sequences operably linked to a polypeptide-encoding segment. A DNA segment is "operably linked" when it is placed into a functional relationship with another DNA segment. For example, a promoter or enhancer is operably linked to a coding sequence if it stimulates the transcription of the sequence. A DNA for a signal sequence is operably linked to a DNA encoding a polypeptide if it is expressed as a preprotein that participates in the secretion of said polypeptide. Generally, DNA sequences that are operably linked are contiguous, and, in the case of a signal sequence, both contiguous and in reading phase. However, an enhancer needs not be contiguous with a coding sequence whose transcription it controls. Linking is accomplished by ligation at convenient restriction sites or at adapters or linkers inserted in lieu thereof. The selection of an appropriate promoter sequence generally depends upon a host cell selected for the expression of a DNA segment. Examples of suitable promoter sequences include prokaryotic, and eukaryotic promoters well known in the art (see, e.g. Sambrook and Russell, 2001, *supra).* A transcriptional regulatory sequence typically includes a heterologous enhancer or promoter that is recognised by the host. The selection of an appropriate promoter depends upon the host, but promoters such as the trp, lac and phage promoters, tRNA promoters and glycolytic enzyme promoters are known and available (see, e.g. Sambrook and Russell, 2001, *supra).* An expression vector includes the replication system and transcriptional and translational regulatory sequences together with the insertion site for a polypeptide encoding segment can be employed. Examples of workable combinations of cell lines and expression vectors are described in Sambrook and Russell (2001, *supra)* and in Metzger et al. (1988) Nature 334: 31-36. For example, a suitable expression vector can be expressed in, yeast, e.g. *S.cerevisiae, e.g.,* insect cells, *e.g.,* Sf9 cells, mammalian cells, *e.g.,* CHO cells and bacterial cells, *e.g., E. coli.* A host cell may thus be a prokaryotic or eukarotic host cell. A host cell may be a host cell that is suitable for culture in liquid or on solid media. A host cell is preferably used in a method for producing a polypeptide of the invention as defined above. A method comprises the step of culturing a host cell under conditions conducive to the expression of a polypeptide. Optionally a method may comprise recovery of a polypeptide. A polypeptide may e.g. be recovered from the culture medium by standard protein purification techniques, including a variety of chromatography methods known in the art per se.

Alternatively, a host cell is a cell that is part of a multicellular organism such as a transgenic plant or animal, preferably a non-human animal. A transgenic plant comprises in at least a part of its cells a vector as defined above. Methods for generating transgenic plants are e.g. described in U.S. 6,359,196 and in the references cited therein. Such transgenic plant may be used in a method for producing a polypeptide of the invention as defined above, said method comprising the step of recovering a part of a transgenic plant comprising in its cells the vector or a part of a descendant of such transgenic plant, whereby said plant part contains a polypeptide, and, optionally recovery of a polypeptide from said plant part. Such method is also described in U.S. 6,359,196 and in the references cited therein. Similarly, a transgenic animal comprises in its somatic and germ cells a vector as defined above. A transgenic animal preferably is a non-human animal. Methods for generating transgenic animals are e.g. described in WO 01/57079 and in the references cited therein. Such transgenic animal may be used in a method for producing a polypeptide of the invention as defined above, said method comprising the step of recovering a body fluid from a transgenic animal comprising a vector or a female descendant thereof, wherein the body fluid contains a polypeptide, and, optionally recovery of a polypeptide from said body fluid. Such methods are also described in WO 01/57079 and in the references cited therein. A body fluid containing a polypeptide preferably is blood or more preferably milk.

Another method for preparing a polypeptide is to employ an *in vitro* transcription/translation system. DNA encoding a polypeptide is cloned into an expression vector as described *supra.* Said expression vector is then transcribed and translated *in vitro.* A translation product can be used directly or first purified. A polypeptide resulting from *in vitro* translation typically does not contain the post-translation modifications present on a polypeptide synthesised *in vivo,* although due to the inherent presence of microsomes some post-translational modification may occur. Methods for synthesis of polypeptides by *in vitro* translation are described by, for example, Berger & Kimmel, Methods in Enzymology, Volume 152, Guide to Molecular Cloning Techniques, Academic Press, Inc., San Diego, CA, 1987.

### Gene therapy

Some aspects of the invention concern the use of a nucleic acid construct or expression vector comprising a nucleotide sequence as defined above, wherein the vector is a vector that is suitable for gene therapy. Vectors that are suitable for gene therapy are described in Anderson 1998, Nature 392: 25-30; Walther and Stein, 2000, Drugs 60: 249-71; Kay et al., 2001, Nat. Med. 7: 33-40; Russell, 2000, J. Gen. Virol. 81: 2573-604; Amado and Chen, 1999, Science 285: 674-6; Federico, 1999, Curr. Opin. Biotechnol.10: 448-53; Vigna and Naldini, 2000, J. Gene Med. 2: 308-16; Marin et al., 1997, Mol. Med. Today 3: 396-403; Peng and Russell, 1999, Curr. Opin. Biotechnol. 10: 454-7; Sommerfelt, 1999, J. Gen. Virol. 80: 3049-64; Reiser, 2000, Gene Ther. 7: 910-3; and references cited therein.

Particularly suitable gene therapy vectors include Adenoviral and Adeno-associated virus (AAV) vectors. These vectors infect a wide number of dividing and non-dividing cell types including neuronal cells. In addition an adenoviral vector is usually capable of high levels of transgene expression. However, because of the episomal nature of the adenoviral and AAV vectors after cell entry, these viral vectors are most suited for therapeutic applications requiring only transient expression of a transgene (Russell, 2000, J. Gen. Virol. 81: 2573-2604; Goncalves, 2005, Virol J. 2(1):43) as indicated above. A preferred adenoviral vector is modified to reduce the host response as reviewed by Russell (2000, *supra).* Method for neuronal gene therapy using a AAV vector is described by Wang et al., 2005, J Gene Med. March 9 (Epub ahead of print), Mandel et al., 2004, Curr Opin Mol Ther. 6(5):482-90, and Martin et al., 2004, Eye 18(11):1049-55. For gene transfer into APC, preferably dendritic cells, an AAV serotype 1, 2 and 5 is an effective vector and therefore a preferred AAV serotype.

A preferred retroviral vector for application in the present invention is a lentiviral based expression construct. Lentiviral vectors have the unique ability to infect non-dividing cells (Amado and Chen, 1999 Science 285: 674-6). Methods for the construction and use of lentiviral based expression constructs are described in U.S. Patent No.'s 6,165,782, 6,207,455, 6,218,181, 6,277,633 and 6,323,031 and in Federico (1999, Curr Opin Biotechnol 10: 448-53) and Vigna et al. (2000, J Gene Med 2000; 2: 308-16). Generally, gene therapy vectors will be as the expression vectors described above in the sense that they comprise a nucleotide sequence encoding a polypeptide of the invention to be expressed, whereby a nucleotide sequence is operably linked to the appropriate regulatory sequences as indicated above. Such regulatory sequence will at least comprise a promoter sequence. A suitable promoter for expression of a nucleotide sequence encoding a polypeptide from gene therapy vectors includes e.g. cytomegalovirus (CMV) intermediate early promoter, viral long terminal repeat promoters (LTRs), such as those from murine moloney leukaemia virus (MMLV) rous sarcoma virus, or HTLV-1 , the simian virus 40 (SV 40) early promoter and the herpes simplex virus thymidine kinase promoter. Suitable neuronal promoters are described above.

Several inducible promoter systems have been described that may be induced by the administration of small organic or inorganic compounds. Such inducible promoters include those controlled by heavy metals, such as the metallothionine promoter (Brinster et al. 1982 Nature 296: 39-42; Mayo et al. 1982 Cell 29: 99-108), RU-486 (a progesterone antagonist) (Wang et al. 1994 Proc. Natl. Acad. Sci. USA 91: 8180-8184), steroids (Mader and White, 1993 Proc. Natl. Acad. Sci. USA 90: 5603-5607), tetracycline (Gossen and Bujard 1992 Proc. Natl. Acad. Sci. USA 89: 5547-5551; U.S. Pat. No. 5,464,758; Furth et al. 1994 Proc. Natl. Acad. Sci. USA 91: 9302-9306; Howe et al. 1995 J. Biol. Chem. 270: 14168-14174; Resnitzky et al. 1994 Mol. Cell. Biol. 14: 1669-1679; Shockett et al. 1995 Proc. Natl. Acad. Sci. USA 92: 6522-6526) and the tTAER system that is based on the multi-chimeric transactivator composed of a tetR polypeptide, as activation domain of VP16, and a ligand binding domain of an estrogen receptor (Yee et al., 2002, US 6,432,705).

Suitable promoters for nucleotide sequences encoding small RNAs for knock down of specific genes by RNA interference (see below) include, in addition to the above mentioned polymerase II promoters, polymerase III promoters. The RNA polymerase III (pol III) is responsible for the synthesis of a large variety of small nuclear and cytoplasmic non-coding RNAs including 5S, U6, adenovirus VA1, Vault, telomerase RNA, and tRNAs. The promoter structures of a large number of genes encoding these RNAs have been determined and it has been found that RNA pol III promoters fall into three types of structures (for a review see Geiduschek and Tocchini-Valentini, 1988 Annu. Rev. Biochem. 57: 873-914; Willis, 1993 Eur. J. Biochem. 212: 1-11; Hernandez, 2001, J. Biol. Chem. 276: 26733-36). Particularly suitable for expression of siRNAs are the type 3 of the RNA pol III promoters, whereby transcription is driven by cis-acting elements found only in the 5'-flanking region, i.e. upstream of the transcription start site. An upstream sequence element includes a traditional TATA box (Mattaj et al., 1988 Cell 55, 435-442), proximal sequence element and a distal sequence element (DSE; Gupta and Reddy, 1991 Nucleic Acids Res. 19, 2073-2075). An example of a gene under the control of the type 3 pol III promoter is a U6 small nuclear RNA (U6 snRNA), 7SK, Y, MRP, H1 and telomerase RNA genes (see e.g. Myslinski et al., 2001, Nucl. Acids Res. 21: 2502-09).

A gene therapy vector may optionally comprise a second or one or more further nucleotide sequence coding for a second or further protein. A second or further protein may be a (selectable) marker protein that allows for the identification, selection and/or screening for a cell containing the expression construct. A suitable marker protein for this purpose is e.g. the fluorescent protein GFP, and the selectable marker genes HSV thymidine kinase (for selection on HAT medium), bacterial hygromycin B phosphotransferase (for selection on hygromycin B), Tn5 aminoglycoside phosphotransferase (for selection on G418), and dihydrofolate reductase (DHFR) (for selection on methotrexate), CD20, the low affinity nerve growth factor gene. Sources for obtaining these marker genes and methods for their use are provided in Sambrook and Russel (2001) "Molecular Cloning: A Laboratory Manual (3rd edition), Cold Spring Harbor Laboratory, Cold Spring Harbor Laboratory Press, New York.

A gene therapy vector is preferably formulated in a pharmaceutical composition comprising a suitable pharmaceutical carrier as defined herein.

### RNA interference

For knock down of or decreasing the expression of a Malt1 polypeptide of the invention, a gene therapy vector or another expression construct is used for the expression of a desired nucleotide sequence that preferably encodes an RNAi agent, i.e. an RNA molecule that is capable of RNA interference or that is part of an RNA molecule that is capable of RNA interference. Such a RNA molecule is referred to as siRNA (short interfering RNA, including e.g. a short hairpin RNA). Alternatively, a siRNA molecule may directly, e.g. in a pharmaceutical composition that is administered to a subject in a need thereof.

A desired nucleotide sequence comprises an antisense code DNA coding for the antisense RNA directed against a region of the target gene mRNA, and/or a sense code DNA coding for the sense RNA directed against the same region of the target gene mRNA. In a DNA construct of the invention, the antisense and sense code DNAs are operably linked to one or more promoters as herein defined above that are capable of expressing the antisense and sense RNAs, respectively. "siRNA" means a small interfering RNA that is a short-length double-stranded RNA that are not toxic in mammalian cells (Elbashir et al., 2001, Nature 411: 494-98; Caplen et al., 2001, Proc. Natl. Acad. Sci. USA 98: 9742-47). The length is not necessarily limited to 21 to 23 nucleotides. There is no particular limitation in the length of siRNA as long as it does not show toxicity. "siRNAs" can be, e.g. at least 15, 18 or 21 nucleotides and up to 25, 30, 35 or 49 nucleotides long. Alternatively, the double-stranded RNA portion of a final transcription product of siRNA to be expressed can be, e.g. at least 15, 18 or 21 nucleotides and up to 25, 30, 35 or 49 nucleotides long.

"Antisense RNA" is an RNA strand having a sequence complementary to a target gene mRNA, and thought to induce RNAi by binding to the target gene mRNA. "Sense RNA" has a sequence complementary to the antisense RNA, and annealed to its complementary antisense RNA to form siRNA. The term "target gene" in this context refers to a gene whose expression is to be silenced due to siRNA to be expressed by the present system, and can be arbitrarily selected. As this target gene, for example, genes whose sequences are known but whose functions remain to be elucidated, and genes whose expressions are thought to be causative of diseases are preferably selected. A target gene may be one whose genome sequence has not been fully elucidated, as long as a partial sequence of mRNA of the gene having at least 15 nucleotides or more, which is a length capable of binding to one of the strands (antisense RNA strand) of siRNA, has been determined. Therefore, genes, expressed sequence tags (ESTs) and portions of mRNA, of which some sequence (preferably at least 15 nucleotides) has been elucidated, may be selected as the "target gene" even if their full length sequences have not been determined.

The double-stranded RNA portions of siRNAs in which two RNA strands pair up are not limited to the completely paired ones, and may contain non-pairing portions due to mismatch (the corresponding nucleotides are not complementary), bulge (lacking in the corresponding complementary nucleotide on one strand), and the like. Non-pairing portions can be contained to the extent that they do not interfere with siRNA formation. The "bulge" used herein preferably comprise 1 to 2 non-pairing nucleotides, and the double-stranded RNA region of siRNAs in which two RNA strands pair up contains preferably 1 to 7, more preferably 1 to 5 bulges. In addition, the "mismatch" used herein is contained in the double-stranded RNA region of siRNAs in which two RNA strands pair up, preferably 1 to 7, more preferably 1 to 5, in number. In a preferable mismatch, one of the nucleotides is guanine, and the other is uracil. Such a mismatch is due to a mutation from C to T, G to A, or mixtures thereof in DNA coding for sense RNA, but not particularly limited to them. Furthermore, in the present invention, the double-stranded RNA region of siRNAs in which two RNA strands pair up may contain both bulge and mismatched, which sum up to, preferably 1 to 7, more preferably 1 to 5 in number. Such non-pairing portions (mismatches or bulges, etc.) can suppress the below-described recombination between antisense and sense code DNAs and make the siRNA expression system as described below stable. Furthermore, although it is difficult to sequence stem loop DNA containing no non-pairing portion in the double-stranded RNA region of siRNAs in which two RNA strands pair up, the sequencing is enabled by introducing mismatches or bulges as described above. Moreover, siRNAs containing mismatches or bulges in the pairing double-stranded RNA region have the advantage of being stable in E. *coli* or animal cells.

The terminal structure of siRNA may be either blunt or cohesive (overhanging) as long as siRNA enables to silence the target gene expression due to its RNAi effect. The cohesive (overhanging) end structure is not limited only to the 3' overhang, and the 5' overhanging structure may be included as long as it is capable of inducing the RNAi effect. In addition, the number of overhanging nucleotide is not limited to the already reported 2 or 3, but can be any numbers as long as the overhang is capable of inducing the RNAi effect. For example, the overhang consists of 1 to 8, preferably 2 to 4 nucleotides. Herein, the total length of siRNA having cohesive end structure is expressed as the sum of the length of the paired double-stranded portion and that of a pair comprising overhanging single-strands at both ends. For example, in the case of 19 bp double-stranded RNA portion with 4 nucleotide overhangs at both ends, the total length is expressed as 23 bp. Furthermore, since this overhanging sequence has low specificity to a target gene, it is not necessarily complementary (antisense) or identical (sense) to the target gene sequence. Furthermore, as long as siRNA is able to maintain its gene silencing effect on the target gene, siRNA may contain a low molecular weight RNA (which may be a natural RNA molecule such as tRNA, rRNA or viral RNA, or an artificial RNA molecule), for example, in the overhanging portion at its one end.

In addition, the terminal structure of the "siRNA" is necessarily the cut off structure at both ends as described above, and may have a stem-loop structure in which ends of one side of double-stranded RNA are connected by a linker RNA (a "shRNA"). The length of the double-stranded RNA region (stem-loop portion) can be, e.g. at least 15, 18 or 21 nucleotides and up to 25, 30, 35 or 49 nucleotides long. Alternatively, the length of the double-stranded RNA region that is a final transcription product of siRNAs to be expressed is, e.g. at least 15, 18 or 21 nucleotides and up to 25, 30, 35 or 49 nucleotides long. Furthermore, there is no particular limitation in the length of the linker as long as it has a length so as not to hinder the pairing of the stem portion. For example, for stable pairing of the stem portion and suppression of the recombination between DNAs coding for the portion, the linker portion may have a clover-leaf tRNA structure. Even though the linker has a length that hinders pairing of the stem portion, it is possible, for example, to construct the linker portion to include introns so that the introns are excised during processing of precursor RNA into mature RNA, thereby allowing pairing of the stem portion. In the case of a stem-loop siRNA, either end (head or tail) of RNA with no loop structure may have a low molecular weight RNA. As described above, this low molecular weight RNA may be a natural RNA molecule such as tRNA, rRNA, snRNA or viral RNA, or an artificial RNA molecule.

To express antisense and sense RNAs from the antisense and sense code DNAs respectively, a DNA construct of the present invention comprises a promoter as defined above. The number and the location of the promoter in a construct can in principle be arbitrarily selected as long as it is capable of expressing antisense and sense code DNAs. As a simple example of a DNA construct of the invention, a tandem expression system can be formed, in which a promoter is located upstream of both antisense and sense code DNAs. This tandem expression system is capable of producing siRNAs having the aforementioned cut off structure on both ends. In the stem-loop siRNA expression system (stem expression system), antisense and sense code DNAs are arranged in the opposite direction, and these DNAs are connected via a linker DNA to construct a unit. A promoter is linked to one side of this unit to construct a stem-loop siRNA expression system. Herein, there is no particular limitation in the length and sequence of the linker DNA, which may have any length and sequence as long as its sequence is not the termination sequence, and its length and sequence do not hinder the stem portion pairing during the mature RNA production as described above. As an example, DNA coding for the above-mentioned tRNA and such can be used as a linker DNA.

In both cases of tandem and stem-loop expression systems, the 5' end may be have a sequence capable of promoting the transcription from the promoter. More specifically, in the case of tandem siRNA, the efficiency of siRNA production may be improved by adding a sequence capable of promoting the transcription from the promoters at the 5' ends of antisense and sense code DNAs. In the case of stem-loop siRNA, such a sequence can be added at the 5' end of the above-described unit. A transcript from such a sequence may be used in a state of being attached to siRNA as long as the target gene silencing by siRNA is not hindered. If this state hinders the gene silencing, it is preferable to perform trimming of the transcript using a trimming means (for example, ribozyme as are known in the art). It will be clear to the skilled person that the antisense and sense RNAs may be expressed in the same vector or in different vectors. To avoid the addition of excess sequences downstream of the sense and antisense RNAs, it is preferred to place a terminator of transcription at the 3' ends of the respective strands (strands coding for antisense and sense RNAs). The terminator may be a sequence of four or more consecutive adenine (A) nucleotides.

### Antibodies

Some aspects of the invention concern the use of an antibody or antibody-fragment that specifically binds to a Malt1 protein as defined above and that is able to inhibit an activity of Malt1 as identified herein. Said antibody is designated as an inhibiting-antibody. Methods for generating antibodies or antibody-fragments that specifically bind to a given polypeptide are described in e.g. Harlow and Lane (1988, Antibodies: A Laboratory Manual, Cold Spring Harbor Laboratory Press, Cold Spring Harbor, NY) and WO 91/19818; WO 91/18989; WO 92/01047; WO 92/06204; WO 92/18619; and US 6,420,113 and references cited therein. The term "specific binding," as used herein, includes both low and high affinity specific binding. Specific binding can be exhibited, e.g., by a low affinity antibody or antibody-fragment having a Kd of at least 10-⁴M. Specific binding also can be exhibited by a high affinity antibody or antibody-fragment, for example, an antibody or antibody-fragment having a Kd of at least about of 10⁻⁷ M, at least 10⁻⁸ M, at least 10⁻⁹ M, at least 10⁻¹⁰ M, or can have a Kd of at least 10⁻¹¹ M or 10⁻¹² M or greater. A preferred assay to assess the inhibition of a Malt1 protein has already be defined earlier.

An antibody is preferably a "human antibody". A human antibody means an antibody in which the variable and constant domain sequences are derived from human sequences. A human antibody provides a substantial advantage in a use of the present invention, as it is expected to minimize the immunogenic and allergic responses that are associated with use of non-human antibodies in a human patient.

For treating a human subject, an antibody would preferably be a chimeric, deimmunised, humanized or human antibody. Such antibodies can reduce immunogenicity and thus avoid human anti-mouse antibody (HAMA) response. It is preferable that the antibody be IgG4, IgG2, or other genetically mutated IgG or IgM which does not augment antibody-dependent cellular cytotoxicity (S. M. Canfield and S. L. Morrison, J. Exp. Med., 1991: 173: 1483-1491) and complement mediated cytolysis (Y. Xu et al., J. Biol. Chem., 1994: 269: 3468-3474; V. L. Pulito et al., J. Immunol., 1996; 156: 2840-2850).

A chimeric antibody may be produced by recombinant processes well known in the art, and has an animal variable region and a human constant region. A humanized antibody usually has a greater degree of human peptide sequences than do chimeric antibodies. In a humanized antibody, only the complementarity determining regions (CDRs), which are responsible for antigen binding and specificity are animal derived and have an amino acid sequence corresponding to the animal antibody, and substantially all of the remaining portions of the molecule (except, in some cases, small portions of the framework regions within the variable region) are human derived and correspond in amino acid sequence to a human antibody (see L. Riechmann et al., Nature, 1988; 332:323-327; G. Winter, United States Patent No. C. Queen et al., U. S. patent number 5,530, 101).

A deimmunised antibody is an antibody in which the T and B cell epitopes have been eliminated, as described in International Patent Application PCT/GB98/01473. They have reduced immunogenicity when applied *in vivo.*

A human antibody can be made by several different ways, including by use of human immunoglobulin expression libraries (Stratagene Corp., La Jolla, California) to produce fragments of human antibodies VH, VL, Fv, Fd, Fab, or (Fab')2, and using these fragments to construct whole human antibodies using techniques similar to those for producing chimeric antibodies. Alternatively, these fragments may be used on their own as inhibitor. Human antibodies can also be produced in transgenic mice with a human immunoglobulin genome. Such mice are available from Abgenix. Inc., Fremont, California, and Medarex, Inc., Annandale, New Jersey.

One can also create single peptide chain binding molecule in which the heavy and light chain Fv regions are connected. Single chain antibodies ("ScFv") and the method of their construction are described in U. S. Patent No. 4,946,778. Alternatively,

Fab can be constructed and expressed by similar means (M. J. Evans et al., J.Immunol. Meth., 1995; 184:123-138). All of the wholly and partially human antibodies are less immunogenic than wholly murine MAbs, and the fragments and single chain antibodies are also less immunogenic. All these types of antibodies are therefore less likely to evoke an immune or allergic response. Consequently, they are better suited for *in vivo* administration in a human subject than wholly animal antibodies, especially when repeated or long-term administration is necessary. In addition, the smaller size of the antibody fragment may help improve tissue bioavailability, which may be critical for better dose accumulation in acute disease indications.

### Peptidomimetics

A small molecule or peptide-like molecule (referred to as peptidomimetics) or a non-peptide molecule that specifically binds to a protein Malt1, that is able to inhibit an activity of said Malt1 and that may be applied in any of the methods of the invention as defined herein as an antagonist of a protein Malt1 of the invention and they may be identified using methods known in the art per se, as e.g. described in detail in US 6,180,084 which incorporated herein by reference. Such methods include e.g. screening libraries of peptidomimetics, peptides, DNA or cDNA expression libraries, combinatorial chemistry and, particularly useful, phage display libraries. These libraries may be screened for an antagonist of a Malt1 protein by contacting the libraries with a substantially purified Malt1 polypeptide of the invention, a fragment thereof or a structural analogue thereof. A preferred assay to assess the inhibition of a Malt1 activity by a peptide-like molecule (referred to as peptidomimetics) or a non-peptide has already be defined earlier.

In this document and in its claims, the verb "to comprise" and its conjugations is used in its non-limiting sense to mean that items following the word are included, but items not specifically mentioned are not excluded. In addition the verb "to consist" may be replaced by "to consist essentially of" meaning that a Malt1 inhibitor, or a composition as defined herein may comprise additional component(s) than the ones specifically identified, said additional component(s) not altering the unique characteristic of the invention. In addition, reference to an element by the indefinite article "a" or "an" does not exclude the possibility that more than one of the element is present, unless the context clearly requires that there be one and only one of the elements. The indefinite article "a" or "an" thus usually means "at least one". The word "about" or "approximately" when used in association with a numerical value (about 10) preferably means that the value may be the given value of 10 more or less 1% of the value.

All patent and literature references cited in the present specification are hereby incorporated by reference in their entirety.

The following examples are offered for illustrative purposes only, and are not intended to limit the scope of the present invention in any way.

### Figure legends

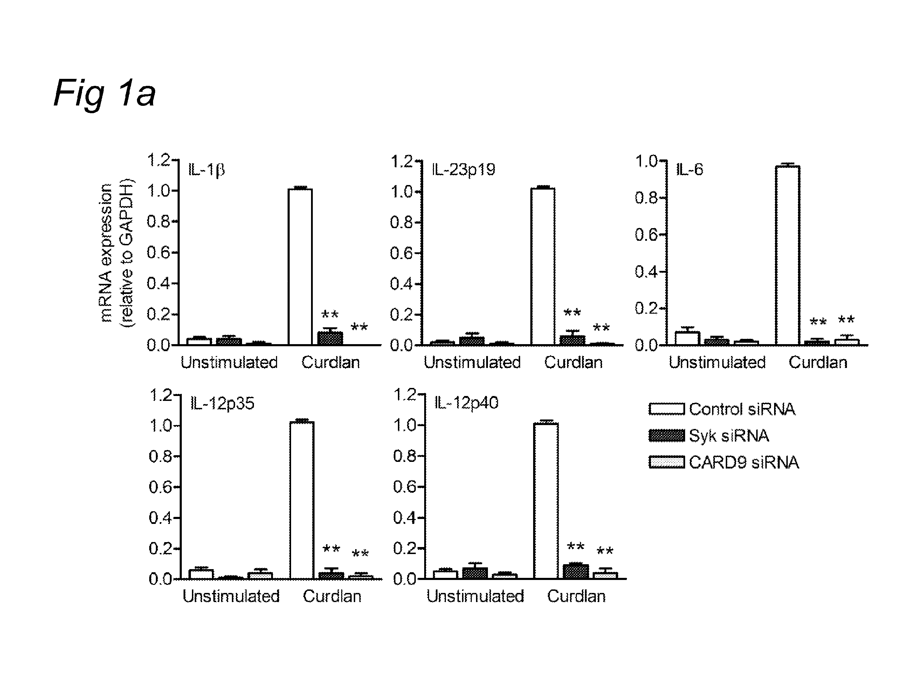

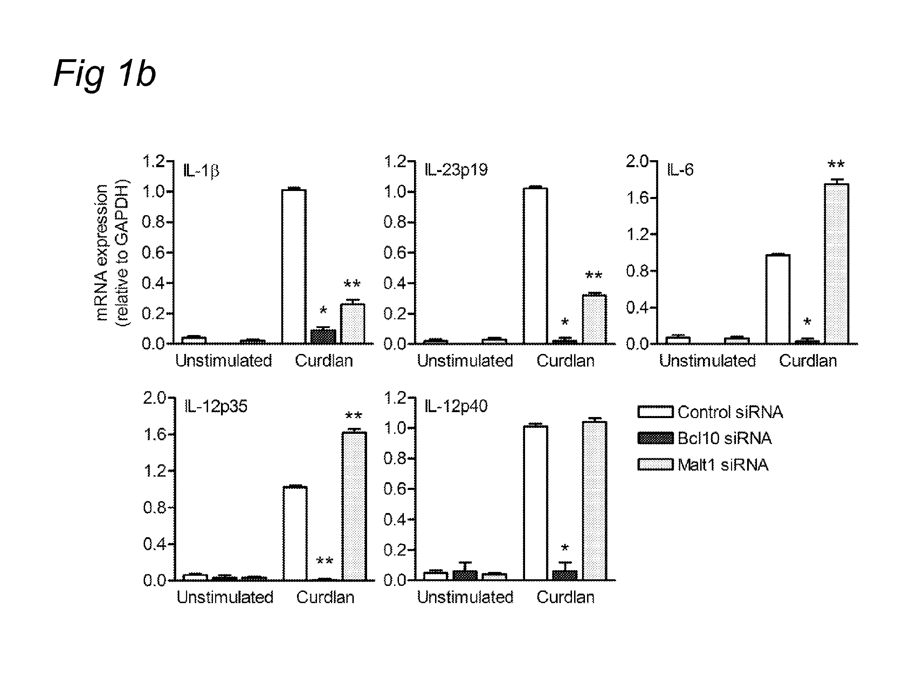

**Figure 1****. Dectin-1-induced cytokine expression requires Syk, CARD9 and Bc110,** whereas Maltl-mediated signaling enhances **IL-1βa**nd IL-23p19, but decreases IL-6 and IL-12p35 expression
   (A and B) Quantitative real-time PCR for indicated mRNAs in curdlan-stimulated DCs after Syk, CARD9 (A), Bc110 and Malt1 (B) silencing by RNA interference (siRNA). Expression is normalized to GAPDH and set at 1 in curdlan-stimulated cells. Data are mean ± s.d. of four independent experiments, ^{*}*p* < 0.05 and ***p* < 0.01 (Student's t-test).

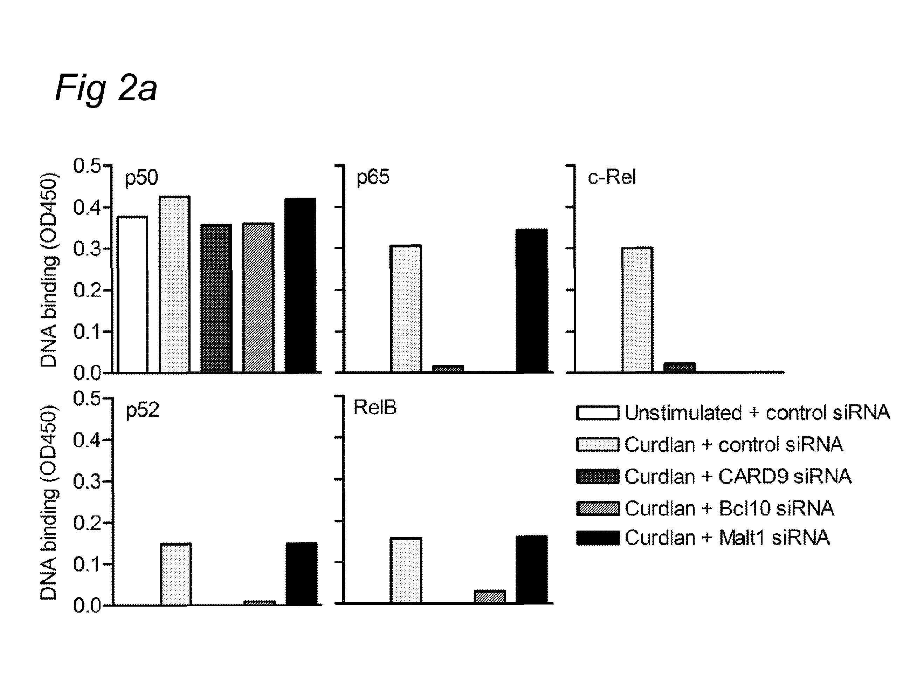

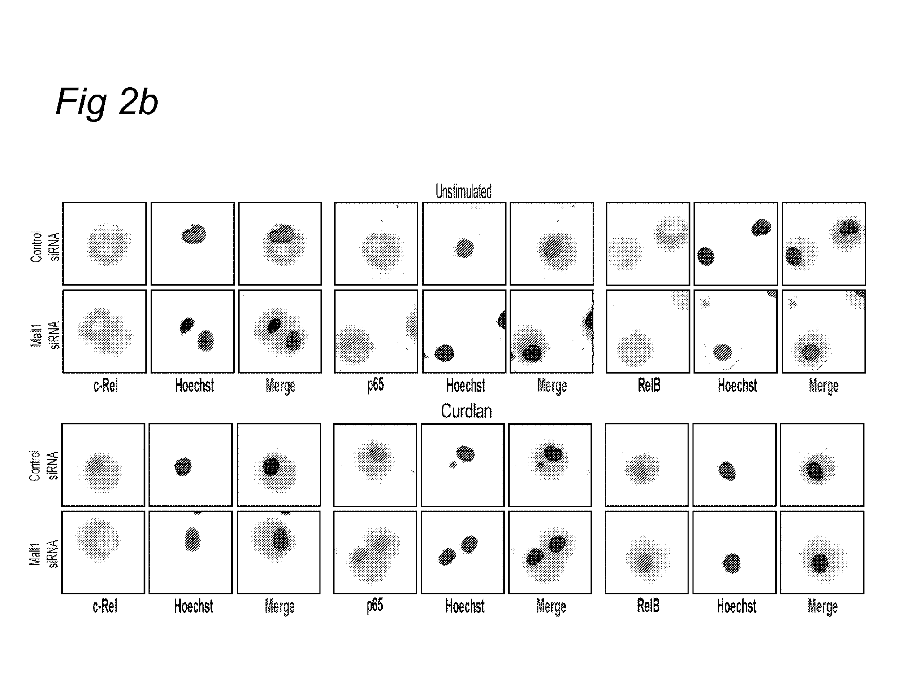

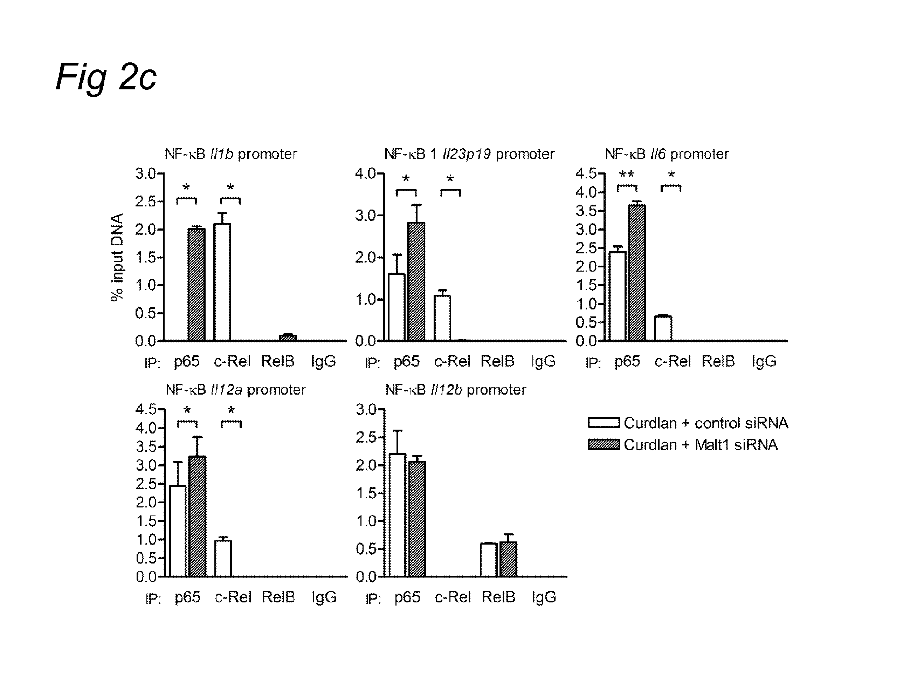

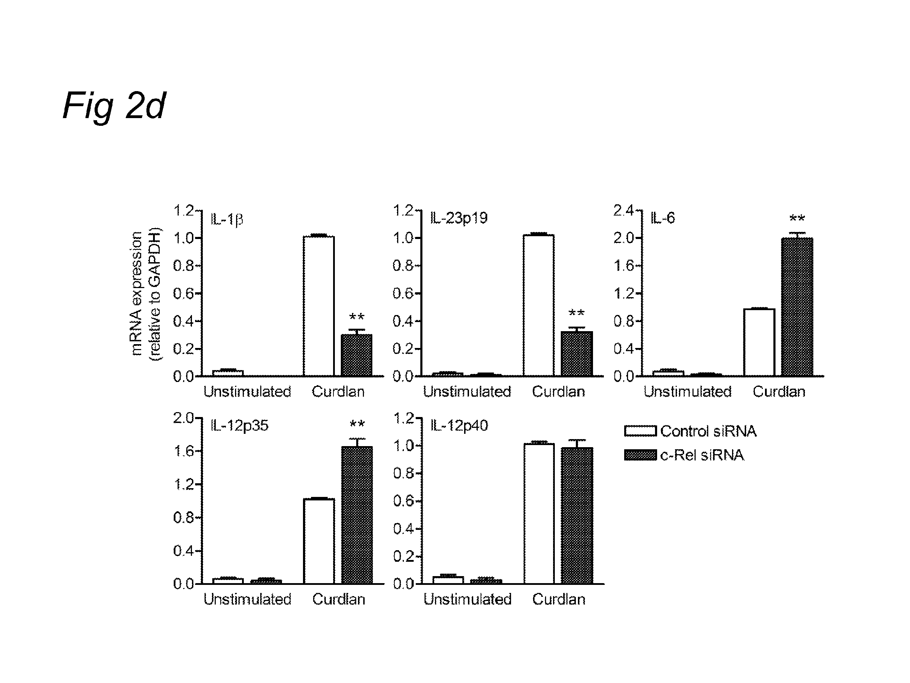

Figure 2. Maltl signaling by dectin-1 is specifically required for c-Rel-dependent cytokine expression
   (A) DNA binding of NF-κB subunits in nuclear extracts of curdlan-stimulated DCs after Malt1 silencing by RNA interference (siRNA). Graphs are representative of three independent experiments.
   (B) Translocation of c-Rel, p65 or RelB (red) into the nucleus (Hoechst staining, blue; colocalization (Merge, pink)) in curdlan-stimulated DCs after Malt1 silencing. Stainings are representative of two independent experiments.
   (C) ChIP assays were performed to determine binding of p65, c-Rel and RelB to NF-κB binding motifs of the *Il1b, Il23p19, Il12a, Il12b* and *I16* promoters. Protein-DNA complexes were immunoprecipitated from sheared chromatin isolated from *para*formaldehyde-fixed curdlan-stimulated DCs after Malt1 silencing by RNA interference (siRNA). Immunoprecipitation with mouse IgG served as a negative control. Quantitative real-time PCR reactions for indicated regions were performed. Levels are normalized with respect to the 'input DNA' sample, which had not undergone immunoprecipitation; results are expressed as the % input DNA. Data are mean ± s.d. of two independent experiments, ^{*}*p* < 0.05 and ***p* < 0.01 (Student's t-test).
   (D) Quantitative real-time PCR for indicated mRNAs in curdlan-stimulated DCs after c-Rel silencing by RNA interference (siRNA). Expression is normalized to GAPDH and set at 1 in curdlan-stimulated cells. Data are mean ± s.d. of four independent experiments, ***p* < 0.01 (Student's *t*-test).

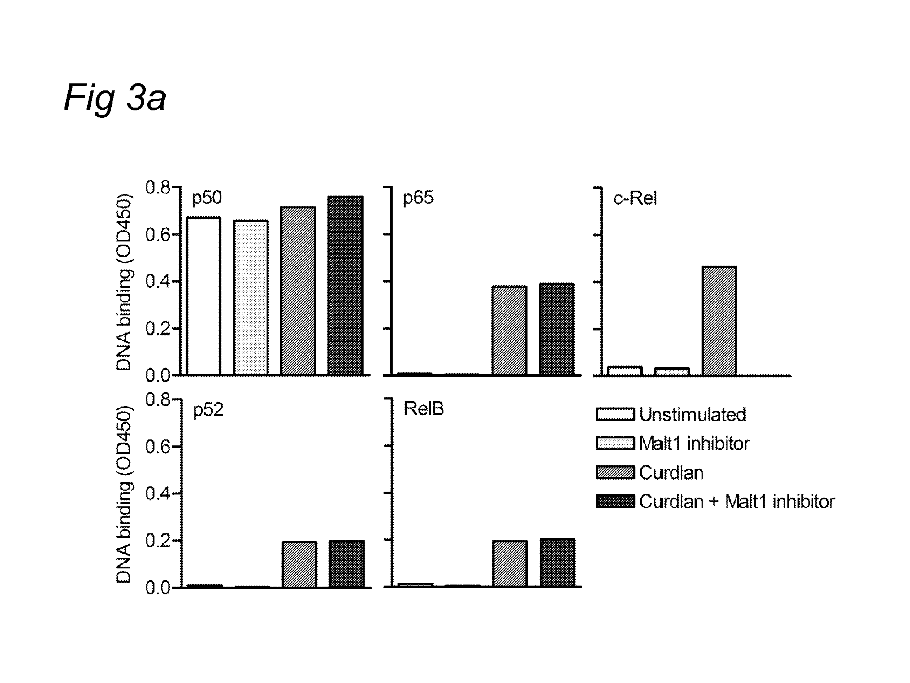

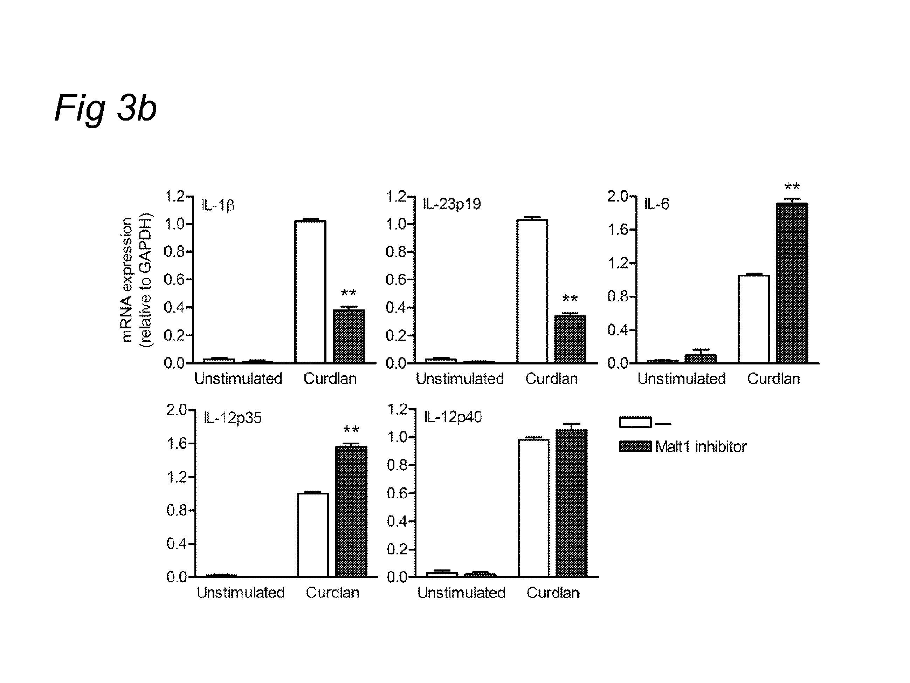

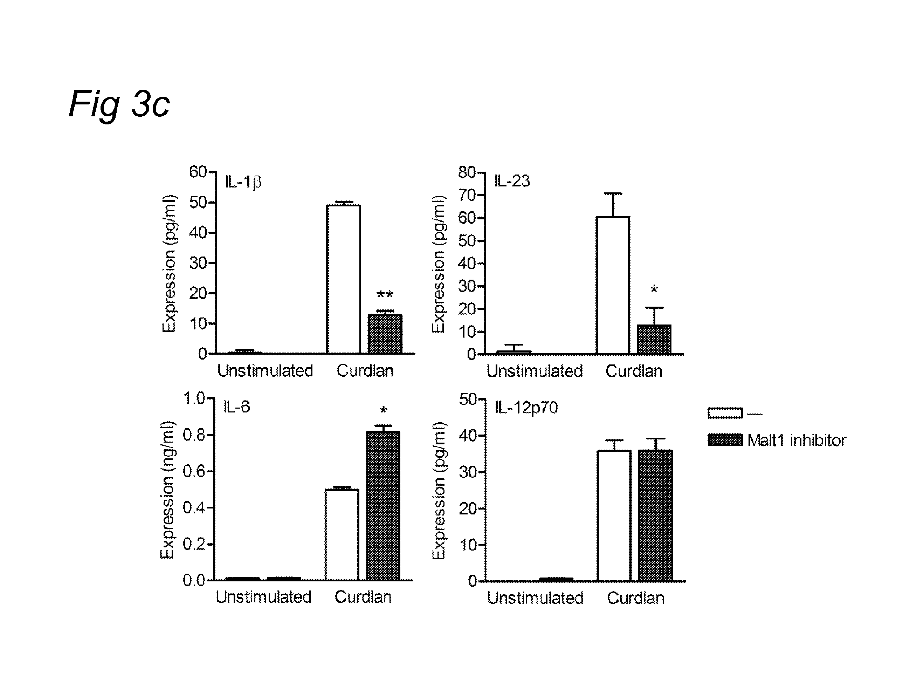

**Figure 3****. Maltl paracaspase activity is required for c-Rel activation and cytokine induction by dectin-1**
   (A) DNA binding of NF-κB subunits in nuclear extracts of curdlan-stimulated DCs after inhibition of Malt1 paracaspase activity by z-VRPR-FMK. Data are representative of three independent experiments.
   (B) Quantitative real-time PCR for indicated mRNAs in curdlan-stimulated DCs after Malt1 paracaspase inhibition. Expression is normalized to GAPDH and set at 1 in curdlan-stimulated cells. Data are mean ± s.d. of three independent experiments, ***p* < 0.01 (Student's t-test).
   (C) Cytokine production was determined by ELISA in supernatants of DCs stimulated with curdlan in the absence or presence of Malt1 paracaspase inhibitor. Data are mean ± s.d. of duplicate cultures, and are representative of five independent experiments, **p* < 0.05 and ***p* < 0.01 (Student's t-test).

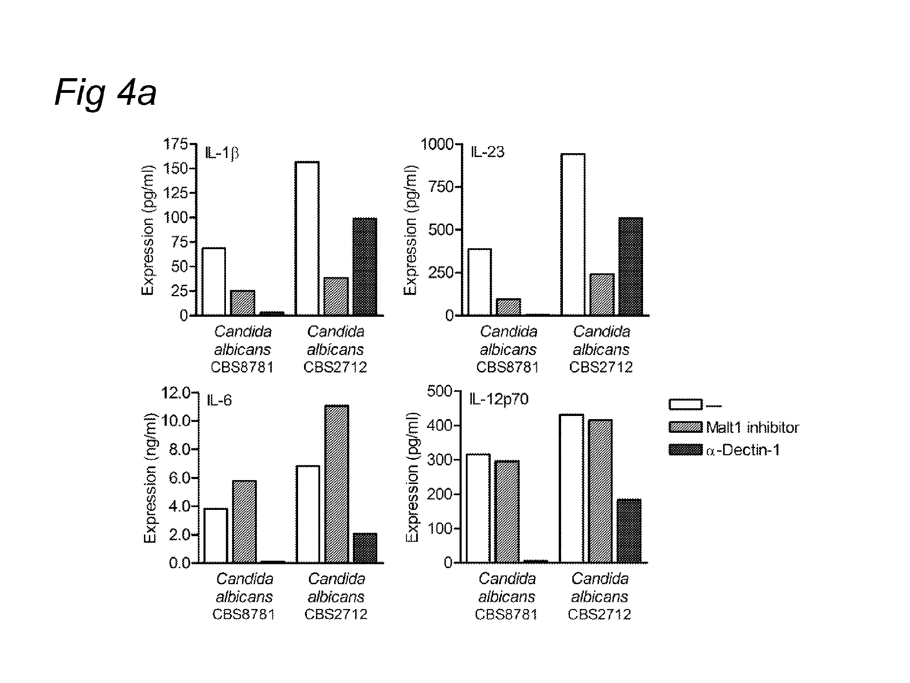

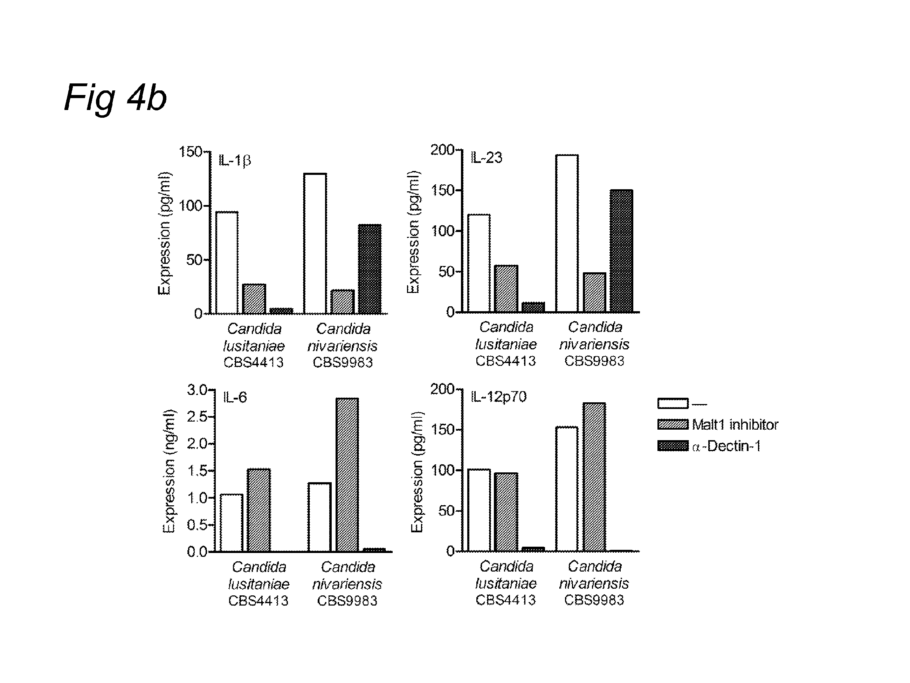

**Figure 4****. Malt activation controls IL-1β and IL-23 production in response to *Candida* spp.**
   (A and B) Cytokine production was determined by ELISA in supernatants of DCs stimulated with *Candida albicans* spp. (A), C. *nivariensis* or C. *lusitaniae* (B) in the absence or presence of Malt1 paracaspase inhibitor z-VRPR-FMK or blocking dectin-1 antibodies. Data are representative of three independent experiments.

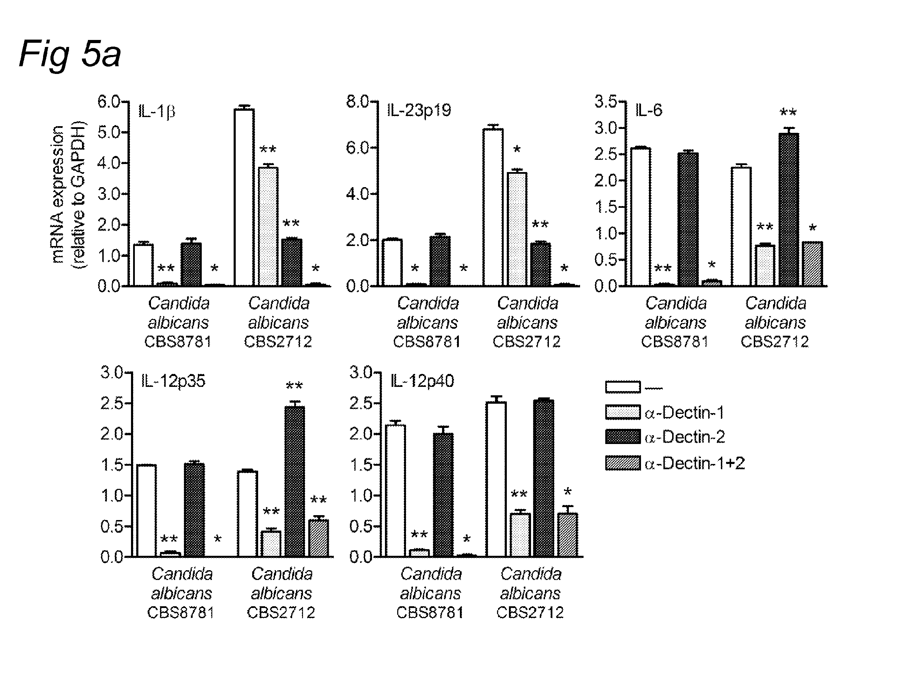

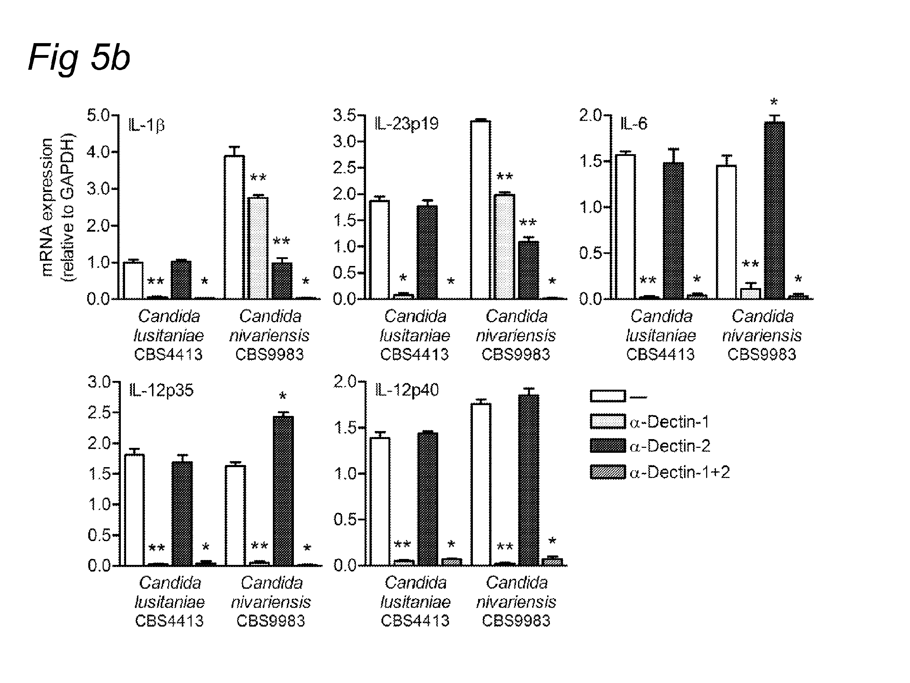

**Figure 5****. Dectin-1 and dectin-2 contribute to *Candida* spp.-induced cytokine expression**
   (A and B) Quantitative real-time PCR for indicated mRNAs in DCs stimulated with *Candida albicans* spp. (A), C. *nivariensis* or C. *lusitaniae* (B) in the absence or presence of blocking antibodies against dectin-1 and/or dectin-2. Expression is normalized to GAPDH and set at 1 in curdlan-stimulated cells. Data are mean ± s.d. of four independent experiments, **p* < 0.05 and ***p* < 0.01 (Student's t-test).

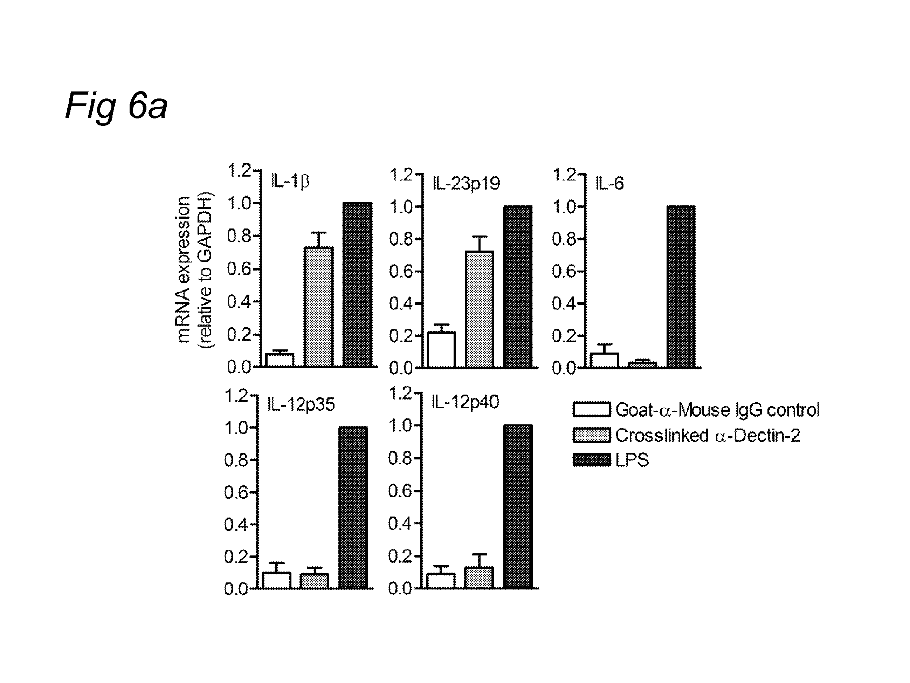

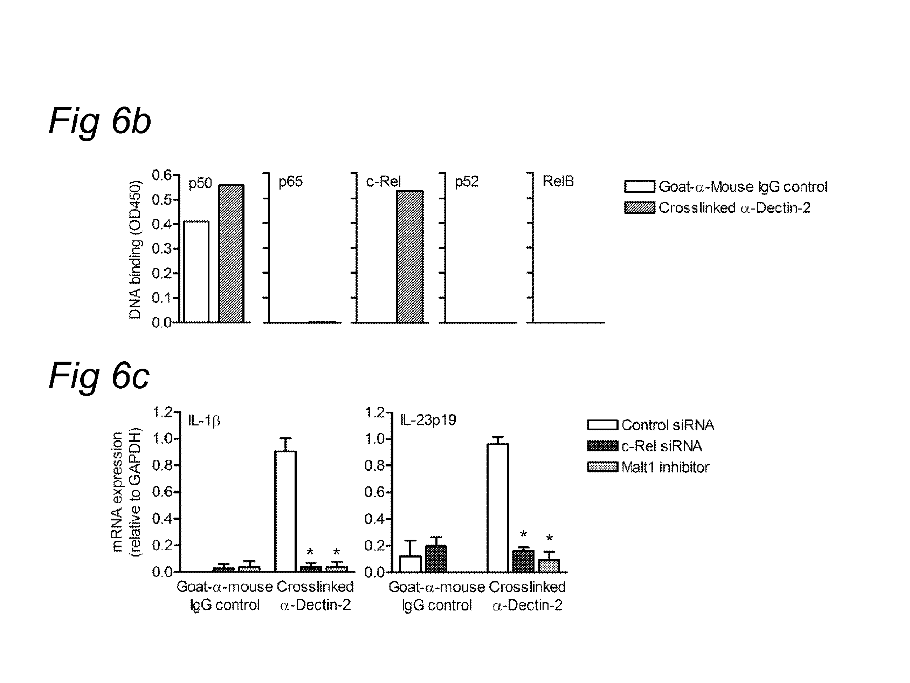

**Figure 6****. Dectin-2 signaling induces Malt1- and c-Rel-dependent IL-1β and IL-23p19 expression**
   (A) Quantitative real-time PCR for indicated mRNAs in DCs stimulated with crosslinked dectin-2 antibodies or LPS. In (A-C), goat-anti-mouse IgG was coated as a control for aspecific activation. Expression is normalized to GAPDH and set at 1 in LPS-stimulated cells. Data are mean ± s.d. of at least four independent experiments.
   (B) DNA binding of NF-κB subunits in nuclear extracts of dectin-2-triggered DCs. Data are representative of two independent experiments.
   (C) Quantitative real-time PCR for indicated mRNAs in DCs stimulated with crosslinked dectin-2 antibodies after c-Rel silencing or Malt1 inhibition with paracaspase inhibitor z-VRPR-FMK. Goat-anti-mouse IgG was coated as a control for aspecific activation. Expression is normalized to GAPDH and set at 1 in LPS-stimulated cells. Data are mean ± s.d. of two independent experiments, **p* < 0.05 (Student's *t*-test).

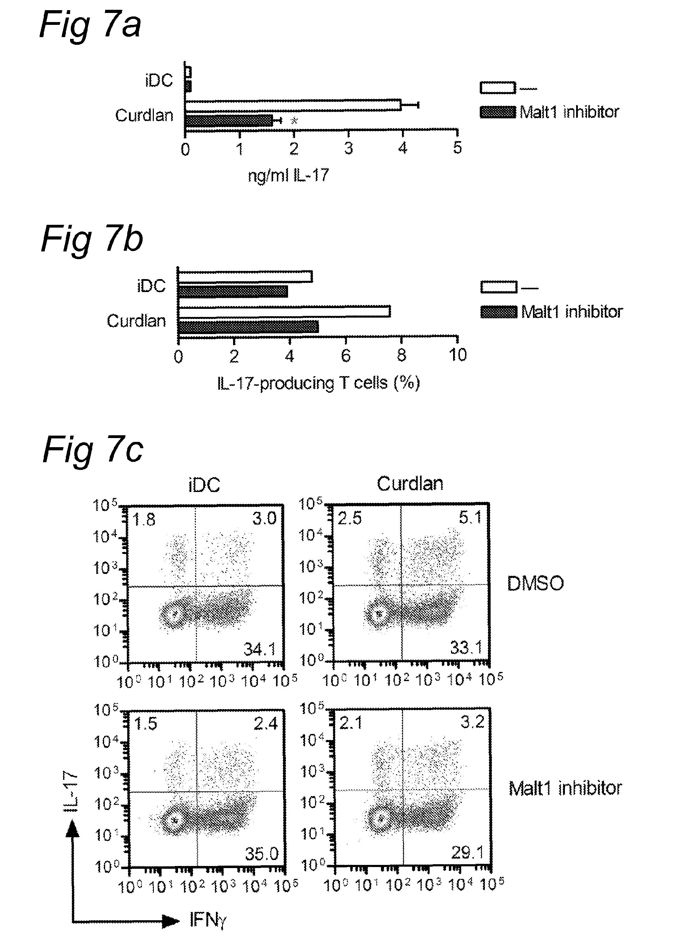

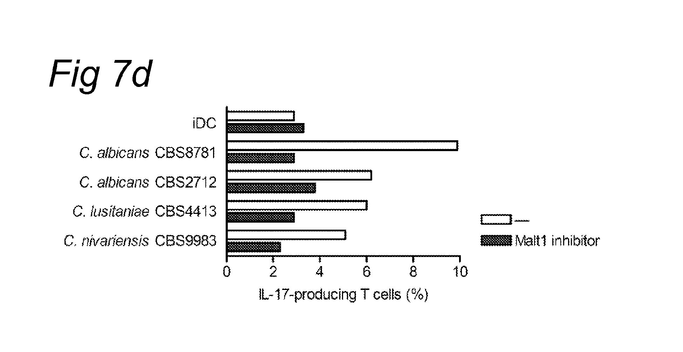

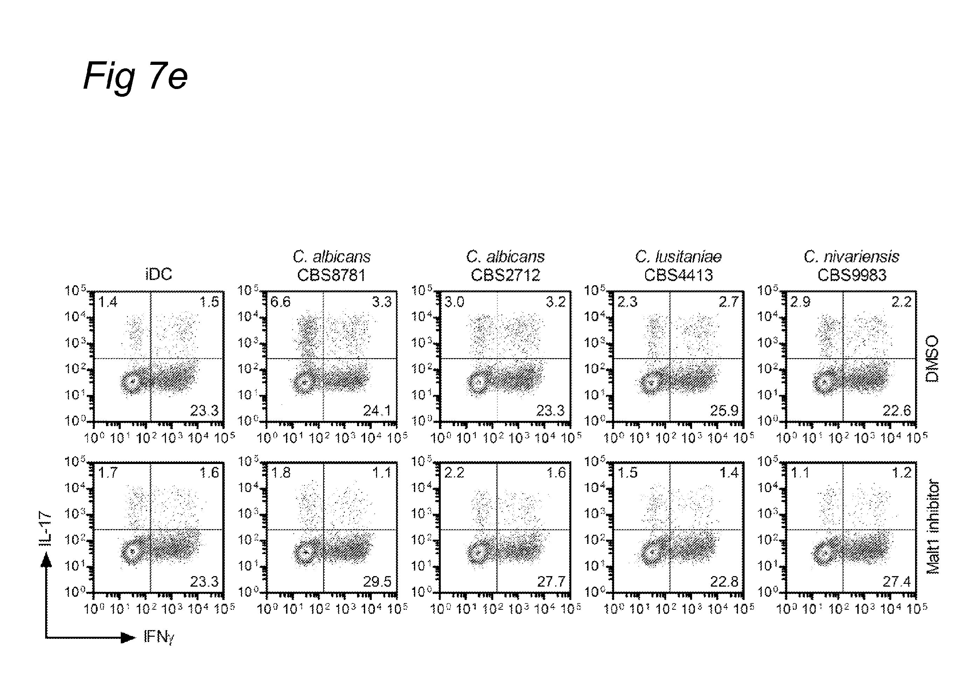

**Figure 7****. Malt1 signaling skews T helper cell polarization towards Th17**
   (A-E) T helper cell polarization was assessed by ELISA by measuring IL-17 production in supernatants at day 5 (A) or by flow cytometry by staining for intracellalur IL-17 or IFNγ expression at day 12 after PMA plus ionomycin restimulation (B-E), after co-culture of memory CD4⁺ T cells with DCs left unstimulated (iDC) or primed with curdlan (A-C) or *Candida* spp. (D and E) in the absence or presence of Malt1 paracaspase inhibitor z-VRPR-FMK. In (B) and (D) the percentage of IL-17-producing T cells are shown, corresponding to the upper left and right quadrants of (C) and (E), respectively. Data are mean ± s.d. of duplicate cultures, **p* < 0.05 (Student's t-test), and are representative of three (A) or two (B-E) independent experiments.

### Examples

### RESULTS

### Dectin-1 signaling via Malt1 affects IL-1β, IL-23p19, IL-6 and IL-12p35 expression

The recruitment of the CARD9-Bc110-Malt1 complex by Syk links dectin-1 on DCs to NF-κB activation, thereby controlling anti-fungal Th17 immunity [10,13-15]. In mice, the pivotal role for Syk and CARD9 in dectin-1 signaling has been established using knock-out models [13,14], while, in contrast, little is known about their role in regulating human adaptive immunity. Here we investigated the role of the CARD9-Bc110-Malt1 module in relaying signals from dectin-1 in human primary DCs to induce cytokine responses. We used the β-glucan curdlan, which is a specific ligand for dectin-1 and induces Syk activation in both mice and human [10,14]. We silenced Syk, CARD9, Bc110 and Malt1 by RNA interference (not shown) and analyzed expression of cytokines involved in Th1 and Th17 polarization. Expression of IL-1β, IL-23p19, IL-6, IL-12p35 and IL-12p40 mRNA was completely abrogated by Syk, CARD9 as well as Bc110 silencing **(****Figure 1A** **and** **1B****).** Notably, Malt1 silencing had distinct effects on the different cytokines; IL-1β and IL-23p19 mRNA expression was strongly decreased, whereas IL-6 and IL-12p35 mRNA was enhanced and IL-12p40 mRNA expression was unaffected by Malt1 silencing **(****Figure 1B****).** TLR4-dependent cytokine expression was unaffected by silencing of either Syk, CARD9, Bc110 or Malt1 (not shown). These data show that Malt1 has a very distinctive function by inducing the T_{H}-17-polarizing cytokines IL-23 and IL-1β, whereas both CARD9 and Bc110 are more generally required for all dectin-1-induced cytokine responses.

### Malt1 controls c-Rel activation by dectin-1

The distinct functions of CARD9, Bc110 and Malt1 in cytokine induction after dectin-1 triggering led us to investigate their functions in the activation of NF-κB. Dectin-1 triggering activates all NF-κB subunits in a Syk-dependent manner, which is crucial to dectin-1-induced cytokine responses [10]. We first determined nuclear translocation and subsequent DNA binding of the different subunits after dectin-1 triggering. NF-κB dimers are normally retained inactive in the cytoplasm and translocate into the nucleus upon activation [21]. In control-silenced DCs, dectin-1 triggering by curdlan resulted in activation of p65, c-Rel, p52 and RelB, while p50 DNA binding could already be detected in unstimulated cells **(****Figure 2A****).** Silencing of either CARD9 or Bc110 in DCs completely impaired activation of p65, c-Rel, RelB and p52 after curdlan stimulation **(****Figure 2A****).** Strikingly, Malt1 silencing specifically abrogated c-Rel activation **(****Figure 2A****),** whereas nuclear translocation of the other subunits was unaffected **(****Figure 2A****).** Immunofluorescence stainings showed that Malt1 silencing interfered with the nuclear translocation of c-Rel but neither with p65 nor RelB **(****Figure 2B****).** These data strongly suggest that Malt1 is required for selective activation of c-Rel-containing NF-κB dimers, whereas recruitment of CARD9 and Bc110 are an absolute requirement for activation of all NF-κB subunits.

### c-Rel activation by Malt1 induces Th17-polarizing cytokines IL-1β and II-23

We next used chromatin immunoprecipitation (ChIP) assays to investigate the effect of Malt1-induced c-Rel activation on the DNA binding of the NF-κB subunits to different cytokine promoters. Our data show that the NF-κB site of the *Il1b* promoter was solely occupied by c-Rel, while both c-Rel and p65 were bound to the *Il23, Il6* and *Il12a* promoters after curdlan stimulation of control-silenced DCs, albeit in different ratios **(****Figure 2C****)**. Malt1 silencing completed abrogated binding of c-Rel to the *Il1b, Il23, Il6* and *Il12a* promoters after dectin-1 triggering **(****Figure 2C****)**, consistent with a pivotal role for Malt1-mediated signaling in c-Rel activation. The absence of c-Rel activation allowed binding of p65 to the promoters as is evident from the higher p65 association with the different promoters **(****Figure 2C****)**. Notably, c-Rel binding to the *Il1b* promoter was completely replaced by p65 binding after Malt1 silencing **(****Figure 2C****).** This suggests that the Il1b promoter is preferentially bound by c-Rel and that c-Rel is a stronger activator *of Il1b* transcription than p65, since c-Rel replacement by p65 after Malt1 silencing resulted in significantly reduced IL-1β expression (Figure 1B). While Malt1 silencing abolished c-Rel binding to both the *Il23* and *Il12a* promoter after dectin-1 triggering, loss of c-Rel activation had opposite effects on *Il23* and *Il12a* transcription as IL-23p19 mRNA levels were severely decreased, while IL-12p35 mRNA was enhanced **(****Figure 1B****)**. These results are consistent with our previous findings showing that c-Rel is a stronger transactivator of *Il23* but a weaker transactivator *of Il12a* transcripton than p65 [10]. IL-6 expression was enhanced after Malt1 silencing **(****Figure 1B****)**, suggesting that c-Rel functions as an inhibitory factor when bound to the *Il6* promoter. The *Il12b* promoter was not bound by c-Rel in either control- or Malt1-silenced cells after curdlan stimulation **(****Figure 2C****)**, consistent with the similar IL-12p40 mRNA levels in both control- and Malt1-silenced cells after dectin-1 triggering **(****Figure 1B****).**

In order to further demonstrate the importance of c-Rel in the transcriptional regulation of the *I*/*1b, Il23, Il6* and *Il12a* genes, we measured cytokine expression in c-Rel-silenced DCs (not shown) after curdlan stimulation (Figure 2D). Similar to Malt1 silencing, c-Rel silencing strongly decreased IL-1β and IL-23p19 mRNA, while enhancing IL-6 and IL-12p35 mRNA levels compared to control-silenced cells after dectin-1 triggering (Figure 2D). IL-12p40 mRNA expression was independent of c-Rel activation **(****Figure 2D****)**, as was LPS-induced cytokine expression (not shown). These results strongly suggest that Malt1-mediated c-Rel activation leads to the expression of IL-1β and IL-23, key cytokines in Th17 differentiation.

### Malt1 proteolytic activity is required for dectin-1-induced c-Rel-dependent cytokine expression

Since Malt1 has paracaspase activity [22,23], we next investigated whether the adaptor or protease function of Malt1 is involved in the selective activation of c-Rel after dectin-1 triggering. We used z-VRPR-FMK, a compound which blocks the proteolytic activity of Malt1 [22]. Inhibition of Malt1 proteolytic activity completely abolished activation of c-Rel without affecting the other NF-κB subunits **(****Figure 3A****)**, which is similar to Malt1 silencing **(****Figure 2A****)**, Immunofluoresence stainings confirmed that Malt1 inhibition specifically interferes with nuclear translocation of c-Rel after curdlan stimulation (not shown). Malt1 paracaspase inhibition also markedly reduced both IL-1β and IL-23p19 mRNA levels and slightly enhanced IL-6 and IL-12p35 mRNA production after curdlan stimulation **(****Figure 3B****)**, similarly to Malt1 silencing **(****Figure 1B****)**. IL-12p40 mRNA production was neither dependent on Malt1 expression nor activation **(****Figure 3B****)**. We next measured cytokine production and found that Malt1 inhibition severely reduced IL-1β and IL-23 protein expression, without affecting IL-12p70 expression and only slightly enhancing IL-6 expression **(****Figure 3C****)**, indicating that dectin-1-induced cytokine expression is primarily regulated at the transcriptional level. These results show that Malt1 protease activity is required for specific c-Rel activation and plays a central role in the induction of Th17-polarizing cytokines by dectin-1.

### Malt1 directs expression of Th17-polarizing cytokines during Candida spp. infection

Dectin-1 plays an important role in anti-fungal immunity through the induction of Th1 and Th17 differentiation [10,14]. Since *Candida albicans* infections are amongst the most common causes of invasive fungal infections in immunocompromised patients [24,25], we used two different C. *albicans* strains to investigate the importance of Malt1 signaling in anti-fungal immune responses. Consistent with its function in the induction of Th17-polarizing cytokines, Malt1 activation is required for expression of IL-1β and IL-23 by DCs in response to both C. *albicans* strain CBS8781 and CBS2712 **(****Figure 4A****)**. As observed with curdlan stimulation, the expression of IL-6 was slightly upregulated as a result of Malt1 protease inhibition, whereas IL-12p70 production was unaffected by Malt1 signaling after C. *albicans* stimulation **(****Figure 4A****)**. To elucidate the contribution of dectin-1 signaling to the Malt1-dependent cytokines responses, we treated DCs with C. *albicans* in the presence of blocking dectin-1 antibodies. Notably, we observed that C. *albicans* CBS8781-induced cytokine expression was completely abrogated after blocking dectin-1, whereas cytokine production after C. *albicans* CBS2712 stimulation was only partially inhibited by dectin-1 antibodies **(****Figure 4A****)**. Malt1 inhibition decreased C. *albicans* CBS2712-induced IL-1β and IL-23 expression more strongly than dectin-1 inhibition **(****Figure 4A****)**. These results suggest that fungal infections trigger not only dectin-1 but also other receptors to induce anti-fungal Th17 responses via Malt1. To further investigate this, we used two different *Candida* species, *C. lusitaniae* and C. *nivarienis,* both emerging pathogenic fungi causing opportunistic infections in transplant and immunocompromised patients [24,26]. C. *lusitaniae* CBS4413 induced cytokine production in a dectin-1-dependent manner, while C. *nivariensis* CBS9983 was only partially dependent on dectin-1 signaling for the production of IL-1β, IL-23, IL-6 and IL-12p70 **(****Figure 4B****)**. Both IL-1β and IL-23 production by C. *lusitaniae* and C. *nivariensis* was largely dependent on Malt1 protease activity **(****Figure 4B****)**. Noteworthy, *Candida* spp. that trigger Malt1 activation via dectin-1 in combination with other unidentified receptor(s) induce higher levels of IL-1β and IL-23 in DCs than those that trigger only dectin-1 **(****Figure 4A** **and** **4B****)**. Similar to the C. *albicans* strains, C. *lusitaniae* and C. *nivariensis* stimulation resulted in slightly enhanced IL-6 expression after Malt1 inhibition, while IL-12p70 was unaffected **(****Figure 4B****)**. These data suggest that c-Rel activation by Malt1 signaling controls anti-fungal Th17 immunity to *Candida* spp.

### Dectin-2 contributes to cytokine response during Candida spp. infection

We next set out to identify the fungal PRR(s) on DCs that are triggered by C. *albicans* CBS2712 and C. *nivariensis* to induce Malt1 activation independently of dectin-1. The C-type lectin dectin-2 has been shown to participate in fungal Th17 immunity in mice [19,20]. We explored a role for dectin-2 in cytokine responses to *Candida* spp. by using blocking antibodies against dectin-2. Notably, induction of IL-1β and IL-23p19 mRNA by both C. *albicans* CBS2712 and C. *nivariensis* was partially abolished by dectin-2 antibodies, while blocking both dectin-1 and dectin-2 completely abrogated expression of IL-1β and IL-23p19 **(****Figure 5****)**. These data strongly suggest that dectin-2 signaling contributes together with dectin-1 to the induction of these Th17-polarizing cytokines by C. *albicans* CBS2712 and C. *nivariensis.* Blocking dectin-2 triggering by C. *albicans* CBS2712 or C. *nivariensis* slightly increased IL-6 but greatly enhanced IL-12p35 mRNA expression **(****Figure 5****),** most likely reflecting the negative influence of c-Rel binding to the respective promoters on *Il6* and *Il12a* transcription. C. *nivariensis-*induced IL-6 and IL-12p35 mRNA expression was dependent on both dectin-1 and dectin-2, while C. *albicans* CBS2712 induced IL-6 and IL-12p35 expression via dectin-1, as blocking both dectin-1 and dectin-2 had no additional effects compared to blocking dectin-1 alone **(****Figure 5****).** IL-12p40 mRNA expression by C. *albicans* CBS2712 and C. *nivariensis* was independent of dectin-2 triggering **(****Figure 5****)**. These data suggest that dectin-2 signaling through Malt1 controls only c-Rel-dependent gene expression without affecting c-Rel-independent transcription. The residual expression of IL-6, IL-12p35 and IL-12p40 induced by C. *albicans* CBS2712 after either blocking dectin-1 or dectin-1 plus dectin-2 suggests additional involvement of other receptors, such as TLRs **(****Figure 5A****)**. As expected, dectin-2 antibodies did not interfere with cytokine expression induced by C. *albicans* CBS8781 and C. *lusitaniae,* since cytokine induction was completely inhibited by blocking dectin-1 antibodies **(****Figure 4** **and** **5****)**. Cytokine protein levels confirmed the mRNA expression data (not shown). Our data demonstrate that both dectin-1 and dectin-2 contribute to anti-fungal Th17-polarizing cytokine responses to various *Candida* spp.

### Malt1 relays dectin-2 signals to induce c-Rel-dependent cytokine expression

We next triggered dectin-2-FcRγ signaling by crosslinking dectin-2 with antibodies and investigated cytokine expression induced by human DCs. Dectin-2 crosslinking induced high levels of IL-1β and IL-23p19 mRNA expression (Figure 6A). Remarkably, dectin-2 crosslinking did neither induce IL-6, IL-12p35 nor IL-12p40 mRNA expression (Figure 6A), consistent with our observations when blocking dectin-2 binding by *Candida* spp. (Figure 5) and strongly suggesting that dectin-2 triggering specifically induces IL-1β and IL-23p19. These results confirm that dectin-1 and dectin-2 signaling converge to boost the expression of Th17-polarizing cytokines, as we observed after *Candida* spp. stimulation.

We next investigated whether dectin-2 crosslinking induces NF-κB activation. Notably, dectin-2 triggering resulted in the specific activation of c-Rel, whereas the other NF-κB subunits p65, RelB and p52 were not activated **(****Figure 6B****)**. Consistently, c-Rel-silenced DCs exhibited a defect in the induction of IL-1β and IL-23p19 mRNA expression after dectin-2 crosslinking **(****Figure 6C****)**. We next silenced Malt1 expression to investigate whether dectin-2-FcRγ signaling, like dectin-1, employs Malt1 to specifically activate c-Rel and induce c-Rel-dependent cytokine expression. Similar to c-Rel silencing, Malt1 silencing completely abolished IL-1β and IL-23p19 mRNA production in response to dectin-2 crosslinking **(****Figure 6C****)**. These data demonstrate that dectin-2 has a specialized function in adaptive immunity and specifically contributes to the induction of IL-1β and IL-23p19, emphasizing the importance of the Malt1-c-Rel activation axis in Th17 immunity.

### Malt1 signaling skews T helper cell polarization towards Th17

Since Malt1 links dectin-1 and dectin-2 to the expression of the Th17-polarizing cytokines IL-1β and IL-23 via the activation of c-Rel, we investigated whether Malt1 activation affects adaptive immunity to *Candida* spp. We first co-cultured curdlan-primed DCs with CD4⁺ T cells and measured IL-17 secretion after 5-12 days of co-culture [7]. Malt1 inhibition markedly reduced the capacity of curdlan-primed DCs to induce IL-17 expression in CD4⁺ T cells **(****Figure 7A, 7B and 7C****)**. Thus, Malt1 activity is essential for the induction of Th17-polarizing cytokines in DCs via dectin-1 triggering and subsequent Th17 skewing. The ability of DCs primed by the different *Candida* spp. to promote IL-17 expression in CD4⁺ T cells was completely blocked when Malt1 activation was inhibited in the DCs **(****Figure 7D** **and** **7E****)**. This effect of Malt1 inhibition on the ability of *Candida-primed* DCs to induce Th17 polarization was irrespective of the involvement of either dectin-1 alone (C. *albicans* CBS8781 and C. *lusitaniae)* or combined dectin-1 and dectin-2 triggering (C. *albicans* CBS2712 and C. *nivariensis),* consistent with a general role for Malt1 in inducing the Th17-polarizing cytokines IL-1β and IL-23. Thus, the marked impact of Malt1 inhibition on IL-1β and IL-23p19 expression in response to fungal infections translates to a block in Th17 immunity. Our data demonstrate that Malt1 signaling to c-Rel activation drives anti-fungal Th17 responses.

### DISCUSSION

C-type lectins are amongst the most important innate receptors on DCs to induce anti-fungal Th17 immunity [5,11,14]. Expression of cytokines upon dectin-1 triggering by fungi requires NF-κB activation through Syk-dependent CARD9-Bc110-Malt1 signaling [13,15]. Here we demonstrate that Malt1 activation by dectin-1 and dectin-2 on human DCs induces the expression of Th17-polarizing cytokines IL-1β and IL-23 through selective activation of the NF-κB subunit c-Rel. c-Rel is crucial for optimal transcription of the *Il1b and Il23p19* genes. Dectin-1-induced activation of p65, RelB and c-Rel is completely dependent on the recruitment of CARD9 and Bc110. Notably, Malt1 through its proteolytic paracaspase activity is specifically involved in activation of c-Rel, but dispensible for p65 and RelB activation. Malt1 activation of c-Rel is similarly essential in the induction of Th17-polarizing cytokines by dectin-2. Notably, dectin-2 signaling, unlike dectin-1, only induces strong c-Rel, but not p65 and RelB activation, strongly suggestive of a specific Th17 polarizing function of dectin-2. Our data further show that the involvement of dectin-1 and detcin-2 in anti-fungal immunity by human DCs depends on the *Candida* species. Our data strongly suggest that dectin-2 is crucial in recognition of some pathogenic *Candida* species to boost dectin-1-induced Th17 responses via Malt1. Thus, Malt1-dependent activation of c-Rel dictates adaptive Th17 immunity to fungi by dectin-1 and dectin-2.

Protective immunity against fungal infections via Th17cellular responses requires the expression of IL-1β, IL-23 and IL-6 by DCs. Here we demonstrated that selective activation of NF-κB family member c-Rel by dectin-1 and dectin-2 signaling in response to fungi was essential to expression of IL-1β and IL-23 and consequently Th17 immunity. Our data showed that loss of c-Rel binding to the *I*/*1b* and *Il23p19* promoters strongly decreased IL-1β and IL-23p19 expression even though p65 bound to the NF-κB binding sites in the absence of c-Rel activation. These data further showed that c-Rel was the stronger activator of *Il1b and* Il23 transcription. Furthermore, c-Rel had an inhibitory effect on *Il6* transcription, although p65-driven IL-6 expression allows for sufficient IL-6 to direct Th17 polarization.

Both dectin-1 and dectin-2 triggering resulted in c-Rel activation and c-Rel-dependent IL-1β and IL-23 expression. Engagement of dectin-1 by fungal ligands leads to phosphorylation of the immunoreceptor tyrosine-based activation motif (ITAM)-like sequence within its cytoplasmic domain [15,27] and subsequent association of the spleen tyrosine kinase Syk. Syk activation by dectin-1 is required for NF-κB activation via the assembly of the CARD9-Bcl10-Malt1 module [13,14]. Unlike dectin-1, dectin-2 requires pairing with the adaptor molecule FcRγ to induce signaling [12,20]. Dectin-2 triggering results in phosphorylation of the ITAM of FcRγ and activation of Syk signaling, which induces cytokine expression [20]. Dectin-2 signaling is CARD9-dependent, however a role for Bc110 and Malt1 remains to be established [20]. In antigen receptor signaling, oligomerization of CARD11 (CARMA1) triggers the formation of a scaffold that physically bridges the CARD 11-Bcl10-Malt1 complex with downstream signaling effectors, such as TRAFs and TAK1, to activate the NF-κB-regulating IKK complex [28]. Here we demonstrated that c-Rel activation by dectin-1 and dectin-2 is completely dependent on Malt1 activation. Malt1 is an unique protein as it is the only human paracaspase known [22,23] and our data showed that its paracaspase activity was essential to the activation of c-Rel by dectin-1 and dectin-2. Malt1 has a distinctive function within the CARD9-Bc110-Malt1 complex induced upon dectin-1 and dectin-2 triggering since silencing of CARD9 and Bcl10 by RNA interference completely abrogated the activation of all NF-κB subunits, while Malt1 silencing selectively abrogated c-Rel activation. It is unclear how Malt1 specifically activates c-Rel. A similar observation has been reported for B cell receptor signaling, which uses the CARD 11-Bcl10-Malt1 complex for NF-κB activation [29], while Malt1 is involved in RelB activation after BAFF stimulation in specific B cell subsets [30]. In T cell receptor signaling, the paracaspase activity of Malt1 partially accounts for the amount of NF-κB activation [22], which might reflect the c-Rel-dependency in T cell receptor responses. Only two substrates for Malt1 are known, its binding partner Bc110 and A20 that functions as an inhibitor of NF-κB activation [22,23]. We showed that dectin-2 signaling only induced strong c-Rel activation, while dectin-1 triggering activated all NF-κB subunits; possibly the differential use of downstream molecules like TRAFs by dectin-1 and dectin-2 might underlie these differences in NF-κB activation.

Crosstalk between signaling pathways triggered by recognition of different PAMPs by various PRRs is essential to the induction of immune responses [5,6,11]. Here we demonstrated that dectin-1 and dectin-2 play distinct roles in immunity to fungi. While dectin-1 triggering induced cytokines involved in promoting both Th1 and Th17 polarization, dectin-2 triggering resulted specifically in IL-1β and IL-23p19 expression, which enhanced IL-1β and IL-23 expression in response to different pathogenic *Candida* spp. This suggests that dectin-1 functions more broadly as an anti-fungal receptor inducing protective immunity, while dectin-2 is more specialized in boosting Th17 cellular responses. Our data also demonstrated that even related pathogenic fungi triggered different sets of PRRs, likely contributing to tailoring of pathogen-specific immunity. C. *albicans* strain CBS8781 and C. *lusitaniae* induced cytokine expression in a dectin-1-dependent manner. In contrast, C. *albicans* strain CBS2712 and C. *nivariensis* triggered both dectin-1 and dectin-2 and showed higher IL-1β and IL-23 responses, strongly suggesting that dectin-1 and dectin-2 signaling pathways converge to enhance Th17 immunity. Other *Candida* species might preferentially trigger dectin-2 but not dectin-1 for IL-1β and IL-23p19 protein expression as shown in the study of Saijo *et al.* [19]. Notably, C. *albicans* CBS2712 also induced dectin-1- and dectin-2-independent expression of IL-6, IL-12p35 and IL-12p40. The contribution of TLR signaling, especially via TLR2, and collaboration with dectin-1 signaling has previously been recognized in cytokine responses in *Candida* infections [11,31]. However, C-type lectin triggering seems to be more specialized in IL-23p19 and IL-1β induction. The situation in mice might be more complex as murine TLRs seem to induce c-Rel activation [32], while human TLRs do not [33]. Our data emphasize that immune responses are tailored not only to pathogens from different species but even within species. Thus, interpretation of data obtained with a single pathogen should be done with caution. Research into the role of dectin-1 in fungal infections using knock-out mice has resulted in conflicting data [34,35] and the use of different yet related fungi might underlie these differences. Genetic variation within the *Candida* clade might not only account for differences in pathogenicity [36] but also for the differential recognition by innate receptors. We have demonstrated here that even closely related C. *albicans* strains trigger different sets of PRRs to activate adaptive immune responses.

Maltl-mediated c-Rel activation might be a general mechanism for induction of protective Th17 immunity against fungi and other microbes, since the Card9-Bc110-Malt1 complex might couple other C-type lectins besides dectin-1 and dectin-2 to NF-κB activation. Furthermore, the carbohydrate specificities of dectin-1 and dectin-2 for β-glucans and high mannoses, respectively, signify their importance in more general anti-fungal immunity against species from the phylum *Ascomycota* that contain mannan, chitin and glucan structures in their cell-wall [37,38]. Many pathogenic ascomycetes such as *Candida* spp., *Aspergillus* spp., *Coccidiodides* spp., *Pneumocystis jirovecii* (previously known as *Pneumocystis carinii), Histoplasma capsulatum, Trichophyton rubrum* and *Microsporum audouinni* have been identified as dectin-1 and/or dectin-2 ligands [12,20,34,35,39-41]. In contrast, the cell-wall of fungi from the phylum *Basidiomycota,* such as *Cryptococcus* and *Malassezia* spp, differs from that of ascomycetes, as it is enfolded in a glucuronic acid-rich carbohydrate capsule or consists of lipophilic structures, respectively [42,43]. Th17 responses to *Cr. neoformans* have been reported [44] but are not mediated by dectin-1 [45].

Thus, dectin-1 and dectin-2 control c-Rel activation distinctively via Malt1 activation to induce IL-1β and IL-23 expression and as such tailor Th17 immune responses against fungal pathogens. Given the pivotal role of Th17 responses not only in protective immunity against fungi but also in the pathology of human autoimmune diseases like Crohn's disease, ulcerative colitis, psoriasis and in vaccine development against tuberculosis [3,46], our results might benefit therapeutic developments as Malt1 presents as a rational target for immunomodulatory drugs.

### MATERIALS AND METHODS

**Cells, stimulation, inhibition and RNA interference.** This study was performed in accordance with the ethical guidelines of the Academic Medical Center. Immature DCs (iDC, day 6 and 7) were generated as described previously [10]. In short, human blood monocytes were isolated from buffy coats by a Ficoll and a Percoll gradient and or CD14 selection by magnetic beads. Monocytes were differentiated into immature DCs in the presence of IL-4 and GM-CSFAt day 6 the phenotype of the cultured DCs was confirmed by flow cytometric analysis. 100.000 DCs were stimulated with 10 µg/ml curdlan (Sigma), heat-killed *Candida* spp. [47] (multiplicity of infection (MOI) 10) and 10 ng/ml LPS from *Salmonella typhosa* (Sigma) in 100-150 µl medium. Dectin-2 triggering was induced by pre-incubating DCs for 2 h at room temperature with 5 µg/ml anti-dectin-2 (MAB3114; R&D Systems), followed by cross-linking on goat-anti-mouse IgG (115-006-0710; Jackson)-coated culture plates. Cells were pre-incubated with blocking antibodies or inhibitor for 2 h with 20 µg/ml anti-dectin-1 (MAB1859; R&D Systems), 20 µg/ml anti-dectin-2 (MAB3114; R&D Systems) or 75 µM z-VRPR-FMK (Malt1 inhibitor [22]; Alexis). DCs were transfected with 25 nM siRNA (SEQ ID NO:5-8) using transfection reagens DF4 (Dharmacon), and used for experiments 72 h after transfection. 'SMARTpool' siRNAs used were: Syk (M-003176-03), CARD9 (M-004400-01), Bcl10 (M-004381-02), Malt1 (M-005936-02), c-Rel (M-004768-01) and non-targeting siRNA (D-001206-13) as a control (Dharmacon). This protocol resulted in nearly 100% transfection efficiency as determined by flow cytometry of cells transfected with siGLO-RISC free-siRNA (D-001600-01) and did not induce IFN responses as determined by quantitative real-time PCR analysis [10]: DCs were lysed after 6 hr of stimulation and mRNA was isolated using the mRNA capture kit (Roche) and cDNA was synthesized with the Reverse transcriptase kit (Promega). PCR amplification was performed with the SYBR green method in an ABI 7900HT sequence detection system (Applied Biosystems).Silencing of expression was verified by real-time PCR and flow cytometry (not shown).

Quantitative real-time PCR. mRNA isolation, cDNA synthesis and PCR amplification with the SYBR green method in an ABI 7500 Fast PCR detection system (Applied Biosystems) were performed as described [10]. Standard conditions according to manufacturer's instructions were used. Specific primers were designed using Primer Express 2.0 (Applied Biosystems; **Supplementary Table 1)**. The Cₜ value is defined as the number of PCR cycles where the fluorescence signal exceeds the detection threshold value. For each sample, the normalized amount of target mRNA was calculated from the obtained Cₜ values for both target and GAPDH mRNA with Nₜ = 2^{Ct(GAPDH)-Ct(target)} The relative mRNA expression was obtained by setting Nₜ in curdlan-or LPS-stimulated samples at 1 within one experiment and for each donor.

**Cytokine production**. Cell culture supernatants were harvested after 28 h of stimulation and concentrations of IL-1β, IL-23, IL-6 (Invitrogen) and IL-12p70 (eBioscience) were determined by ELISA.

**Chromatin immunoprecipitation (ChIP) assay**. ChIP assays were performed using the ChIP-IT Express Enzymatic kit (Active Motif) to determine occupancy of the regulatory regions of the *I*/*1b, Il23p19, Il6, Il12a* and *Il12b* promoters by NF-κB as described by the manufacturer. Protein/DNA complexes were immunoprecipitated using anti-p65 (3034; Cell Signaling), anti-c-Rel (4727; Cell Signaling), anti-RelB (4954; Cell Signaling) or negative control IgG (53010; Active Motif), and protein G-coated magnetic beads. DNA was purified after reversal of crosslinks and real-time PCR reactions were then performed with primer sets spanning the NF-κB binding sites (**Supplementary Table 1)**. Primers spanning genomic DNA at cytogenetic location 12 p13.3 (Active Motif) were used as a negative control. To normalize for DNA input, a sample for each condition was taken along which had not undergone immunoprecipitation with a specific antibody ('input DNA'); the results are expressed as the % input DNA.

### Supplementary table 1:

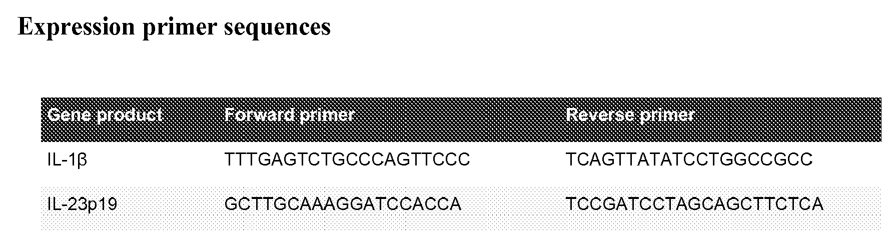

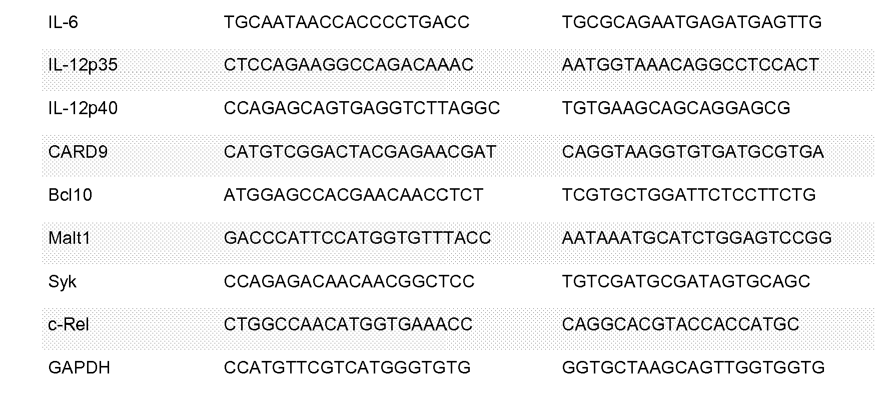

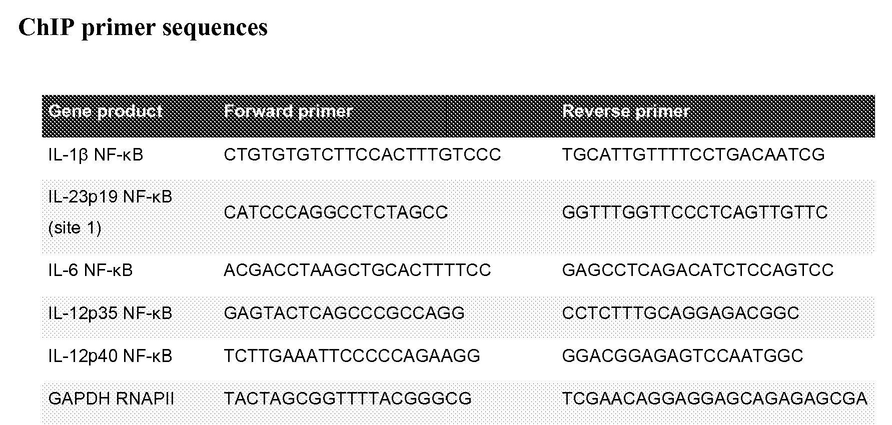

### (SEQ ID NO: 9-42)

Immunofluorescence staining. Stainings were performed as described previously [10]. Briefly, DCs were incubated with stimuli for 2 hours, fixed by paraformaldehyde and incubated with anti-p65, anti-c-Rel or anti-RelB (all from Cell Signaling) followed by Alexa Fluor 594-conjugated goat anti-rabbit (A1 1072; Molecular Probes).

NF-κB DNA binding. Nuclear extracts of DCs were prepared using NucBuster protein extraction kit (Novagen) and NF-κB DNA binding determined using TransAM NF-κB family kit (Active Motif).

Th17 **polarization assay**. Memory CD4⁺ T cells were isolated as described previously [7]. Human PBMC were isolated from heparinized human peripheral blood by density gradient centrifugation on Lymphoprep (Nycomed), and PBMC were subsequently seperated in monocytes and PBL by density gradient centrifugation on Percoll (Pharmacia). Memory CD4⁺ T cells were isolated with CD45R0⁺-PE (Dakopatts) and anti-PE-beads (MACS). iDCs were preincubated for 2 h with inhibitors, activated for 16 h with curdlan or heat-killed *Candida* spp. and subsequently co-cultured with memory CD4⁺ T cells as described (20,000 T cells/2000 DCs in the presence of 10 pg/ml *Staphylococcus aureus* enterotoxin B (Sigma) [7]. After 5 days of co-culture, supernatants were harvested and analyzed for IL-17 production by ELISA (Biosource). Cells were further cultured in the presence of 10 U/ml IL-2 (Chiron) and resting cells were restimulated after 12 days with 100 ng/ml PMA (Sigma) and 1 µg/ml ionomycin (Sigma) for 6 h, the last 5 h in the presence of 10 µg/ml brefeldin A (Sigma), and analyzed for intracellular cytokine expression by staining with biotinylated mouse anti-IL-17 (ebio64DE; eBioscience), followed by incubation with streptavidin-PE (BD Pharmingen) and FITC-conjugated mouse anti-IFN-γ (25723.11; BD).

**Statistical analysis**. Student's t-test for paired observations was used for statistical analyses. Statistical significance was set at a P values of less than 0.05.

### Reference list

1. Romani L (2008) Cell mediated immunity to fungi: a reassessment. Med Mycol 46: 515-529.
2. Ouyang W, Kolls JK, Zheng Y (2008) The biological functions of T helper 17 cell effector cytokines in inflammation. Immunity 28: 454-467.
3. Louten J, Boniface K, de Waal Malefyt R (2009) Development and function of Th17 cells in health and disease. J Allergy Clin Immunol 123: 1004-1011.
4. Romani L (2004) Immunity to fungal infections. Nat Rev Immunol 4: 11-23.
5. Geijtenbeek TBH, Gringhuis SI (2009) Signalling through C-type lectin receptors: shaping immune responses. Nat Rev Immunol 9: 465-479.
6. Medzhitov R (2007) Recognition of microorganisms and activation of the immune response. Nature 449: 819-826.
7. van Beelen AJ, Zelinkova Z, Taanman-Kueter EW, Muller FJ, Hommes DW et al. (2007) Stimulation of the intracellular bacterial sensor NOD2 programs dendritic cells to promote interleukin-17 production in human memory T cells. Immunity 27: 660-669.
8. Weaver CT, Hatton RD (2009) Interplay between the Th17 and TReg cell lineages: a (co-)evolutionary perspective. Nat Rev Immunol 9: 883-889.
9. Murphy KM, Reiner SL (2002) The lineage decisions of helper T cells. Nat Rev Immunol 2: 933-944.
10. Gringhuis SI, den Dunnen J, Litjens M, van der Vlist M, Wevers B et al. (2009) Dectin-1 directs T helper cell differentiation by controlling noncanonical NF-[kappa]B activation through Raf-1 and Syk. Nat Immunol 10: 203-213.
11. Netea MG, Brown GD, Kullberg BJ, Gow NAR (2008) An integrated model of the recognition of Candida albicans by the innate immune system. Nat Rev Micro 6: 67-78.
12. Sato K, Yang XL, Yudate T, Chung JS, Wu J et al. (2006) Dectin-2 is a pattern recognition receptor for fungi that couples with the Fc receptor gamma chain to induce innate immune responses. J Biol Chem 281: 38854-38866.
13. Gross O, Gewies A, Finger K, Schafer M, Sparwasser T et al. (2006) Card9 controls a non-TLR signalling pathway for innate anti-fungal immunity. Nature 442: 651-656.
14. LeibundGut-Landmann S, Gross O, Robinson MJ, Osorio F, Slack EC et al. (2007) Syk- and CARD9-dependent coupling of innate immunity to the induction of T helper cells that produce interleukin 17. Nat Immunol 8: 630-638.
15. Rogers NC, Slack EC, Edwards AD, Nolte MA, Schulz O et al. (2005) Syk-dependent cytokine induction by Dectin-1 reveals a novel pattern recognition pathway for C type lectins. Immunity 22: 507-517.
16. Glocker EO, Hennigs A, Nabavi M, Schaffer AA, Woellner C et al. (2009) A homozygous CARD9 mutation in a family with susceptibility to fungal infections. N Engl J Med 361: 1727-1735.
17. Ferwerda B, Ferwerda G, Plantinga TS, Willment JA, van Spriel AB et al. (2009) Human dectin-1 deficiency and mucocutaneous fungal infections. N Engl J Med 361: 1760-1767.
18. McGreal EP, Rosas M, Brown GD, Zamze S, Wong SY et al. (2006) The carbohydrate-recognition domain of Dectin-2 is a C-type lectin with specificity for high mannose. Glycobiology 16: 422-430.
19. Saijo S, Ikeda S, Yamabe K, Kakuta S, Ishigame H et al. (2010) Dectin-2 Recognition of [alpha]-Mannans and Induction of Th17 Cell Differentiation Is Essential for Host Defense against Candida albicans. Immunity 32: 681-691.
20. Robinson MJ, Osorio F, Rosas M, Freitas RP, Schweighoffer E et al. (2009) Dectin-2 is a Syk-coupled pattern recognition receptor crucial for Th17 responses to fungal infection. J Exp Med 206: 2037-2051.
21. Hayden MS, Ghosh S (2008) Shared principles in NF-[kappa]B signaling. Cell 132: 344-362.
22. Rebeaud F, Hailfinger S, Posevitz-Fejfar A, Tapernoux M, Moser R et al. (2008) The proteolytic activity of the paracaspase MALT1 is key in T cell activation. Nat Immunol 9: 272-281.
23. Coornaert B, Baens M, Heyninck K, Bekaert T, Haegman M et al. (2008) T cell antigen receptor stimulation induces MALT1 paracaspase-mediated cleavage of the NF-[kappa]B inhibitor A20. Nat Immunol 9: 263-271.
24. Butler G, Rasmussen MD, Lin MF, Santos MA, Sakthikumar S et al. (2009) Evolution of pathogenicity and sexual reproduction in eight Candida genomes. Nature 459: 657-662.
25. Pfaller MA, Diekema DJ (2007) Epidemiology of invasive candidiasis: a persistent public health problem. Clin Microbiol Rev 20: 133-163.
26. Borman AM, Petch R, Linton CJ, Palmer MD, Bridge PD et al. (2008) Candida nivariensis, an emerging pathogenic fungus with multidrug resistance to antifungal agents. J Clin Microbiol 46: 933-938.
27. Underhill DM, Rossnagle E, Lowell CA, Simmons RM (2005) Dectin-1 activates Syk tyrosine kinase in a dynamic subset of macrophages for reactive oxygen production. Blood 106: 2543-2550.
28. Rawlings DJ, Sommer K, Moreno-Garcia ME (2006) The CARMA1 signalosome links the signalling machinery of adaptive and innate immunity in lymphocytes. Nat Rev Immunol 6: 799-812.
29. Ferch U, Buschenfelde CMz, Gewies A, Wegener E, Rauser S et al. (2007) MALT1 directs B cell receptor-induced canonical nuclear factor-[kappa]B signaling selectively to the c-Rel subunit. Nat Immunol 8: 984-991.
30. Tusche MW, Ward LA, Vu F, McCarthy D, Quintela-Fandino M et al. (2009) Differential requirement of MALT 1 for BAFF-induced outcomes in B cell subsets. J Exp Med 206: 2671-2683.
31. Gantner BN, Simmons RM, Canavera SJ, Akira S, Underhill DM (2003) Collaborative induction of inflammatory responses by dectin-1 and Toll-like receptor 2. J Exp Med 197: 1107-1117.
32. Saccani S, Pantano S, Natoli G (2003) Modulation ofNF-kappaB activity by exchange of dimers. Mol Cell 11: 1563-74.
33. Gringhuis SI, den Dunnen J, Litjens M, Hof BV, van Kooyk Y et al. (2007) C-type lectin DC-SIGN modulates toll-like receptor signaling via Raf- 1 kinase-dependent acetylation of transcription factor NF-kappa B. Immunity 26: 605-616.
34. Saijo S, Fujikado N, Furuta T, Chung SH, Kotaki H et al. (2007) Dectin-1 is required for host defense against Pneumocystis carinii but not against Candida albicans. Nat Immunol 8: 39-46.
35. Taylor PR, Tsoni SV, Willment JA, Dennehy KM, Rosas M et al. (2007) Dectin-1 is required for beta-glucan recognition and control of fungal infection. Nat Immunol 8: 31-38.
36. Hertz-Fowler C, Pain A (2009) Unity in diversity: lessons from Candida. Nat Rev Micro 7: 763.
37. Chaffin WL, Lopez-Ribot JL, Casanova M, Gozalbo D, Martinez JP (1998) Cell wall and secreted proteins of Candida albicans: identification, function, and expression. Microbiol Mol Biol Rev 62: 130-180.
38. Bernard M, Latge JP (2001) Aspergillus fumigatus cell wall: composition and biosynthesis. Med Mycol 39 Suppl 1: 9-17.
39. Hohl TM, Van Epps HL, Rivera A, Morgan LA, Chen PL et al. (2005) Aspergillus fumigatus triggers inflammatory responses by stage-specific beta-glucan display. PLoS Pathog 1: e30.
40. Sorgi CA, Secatto A, Fontanari C, Turato WM, Belanger C et al. (2009) Histoplasma capsulatum cell wall {beta} -glucan induces lipid body formation through CD18, TLR2, and Dectin-1 receptors: correlation with leukotriene B4 generation and Role in HIV-1 Infection. J Immunol 182: 4025-4035.
41. Viriyakosol S, Fierer J, Brown GD, Kirkland TN (2005) Innate immunity to the pathogenic fungus Coccidioides posadasii is dependent on Toll-like receptor 2 and dectin-1. Infect Immun 73: 1553-1560.
42. Ashbee HR, Evans EG (2002) Immunology of diseases associated with Malassezia species. Clin Microbiol Rev 15: 21-57.
43. Bose I, Reese AJ, Ory JJ, Janbon G, Doering TL (2003) A Yeast under cover: the capsule of Cryptococcus neoformans. Eukaryot Cell 2: 655-663.
44. Kleinschek MA, Muller U, Brodie SJ, Stenzel W, Kohler G et al. (2006) IL-23 enhances the inflammatory cell response in Cryptococcus neoformans infection and induces a cytokine pattern distinct from IL-12. J Immunol 176: 1098-1106.
45. Nakamura K, Kinjo T, Saijo S, Miyazato A, Adachi Y et al. (2007) Dectin-1 is not required for the host defense to Cryptococcus neoformans. Microbiol Immunol 51: 1115-1119.
46. Cooper AM, Khader SA (2008) The role of cytokines in the initiation, expansion, and control of cellular immunity to tuberculosis. Immunol Rev 226: 191-204.
47. de Jong MA, Vriend LE, Theelen B, Taylor ME, Fluitsma D et al. (2010) C-type lectin Langerin is a beta-glucan receptor on human Langerhans cells that recognizes opportunistic and pathogenic fungi. Mol Immunol 47: 1216-1225.

## Claims

1. A Malt1 inhibitor for preventing, delaying and/or treating a Th17-mediated disease.

2. A Malt1 inhibitor according to claim 1 wherein the Th17-mediated disease is a Th17-mediated autoimmune disease and/or a skin disease.

3. A Malt1 inhibitor according to claim 2, wherein the Th17-mediated disease is selected from: Crohn's disease also called inflammatory bowel disease, a skin disease as Psoriasis and/or eczema or as caused by a fungus and/or a bacterium.

4. A Malt1 inhibitor according to any one of claims 1 to 3, wherein said inhibitor inhibits the activation of c-Rel.

5. A Malt1 inhibitor according to any one of claims 1 to 4, wherein said inhibitor is a DNA/RNA molecule, an antibody, a peptide or a chemical compound.

6. A Malt1 inhibitor according to claim 5, which is a peptide derivate Z-VRPR-FMK, Z-LSSR-CHO, Z-LSSR-CMK, Z-GASR-CHO or Z-GASR-CMK, or the chemical compound 5-{[5-(3-chloro-4-methylphenyl)-2- furyl]methylene}-2-thioxodihydro-4,6(1 H,5H)-pyrimidinedione.

7. A composition comprising a Malt1 inhibitor as defined in any one of the preceding claims.

8. Use of a Malt1 inhibitor as defined in any one of claims 1 to 6 or a composition as defined in claim 7 for the manufacture of a medicament for preventing, delaying and/or treating a Th17-mediated disease.

9. A method for preventing, delaying and/or treating a Th17-mediated disease wherein use is made of a Malt1 inhibitor as defined in any one of claims 1 to 6 or a composition as defined in claim 7.

10. A method for identification of a compound inhibiting Malt1, the method comprising the steps of:
a) providing a test cell population capable of expressing a Malt1;
b) contacting the test cell population with the compound;
c) determining an activity of Malt1 in the test cell population contacted with the compound;
d) comparing the activity level determined in (c) with the corresponding activity in a test cell population that is not contacted with the compound; and,
e) identifying a compound that produces a difference in activity of Malt1, between the test cell population that is contacted with the compound and the test cell population that is not contacted with the compound.


SEQUENCE LISTING

<110>  Academisch Medisch Centrum bij de Universiteit van
       Amsterdam

<120>  Treatment of Th17-mediated diseases

<130>  P6033782EP

<160>  42

<170>  PatentIn version 3.3

<210>  1
<211>  2442
<212>  DNA
<213>  homo sapiens

<400>  1
atgtcgctgt tgggggaccc gctacaggcc ctgccgccct cggccgcccc cacggggccg      60

ctgctcgccc ctccggccgg cgcgaccctc aaccgcctgc gggagccgct gctgcggagg     120

ctcagcgagc tcctggatca ggcgcccgag ggccggggct ggaggagact ggcggagctg     180

gcggggagtc gcgggcgcct ccgcctcagt tgcctagacc tggagcagtg ttctcttaag     240

gtactggagc ctgaaggaag ccccagcctg tgtctgctga agttaatggg tgaaaaaggt     300

tgcacagtca cagaattgag tgatttcctg caggctatgg aacacactga agttcttcag     360

cttctcagcc ccccaggaat aaagattact gtaaacccag agtcaaaggc agtcttggct     420

ggacagtttg tgaaactgtg ttgccgggca actggacatc cttttgttca atatcagtgg     480

ttcaaaatga ataaagagat tccaaatgga aatacatcag agcttatttt taatgcagtg     540

catgtaaaag atgcaggctt ttatgtctgt cgagttaata acaatttcac ctttgaattc     600

agccagtggt cacagctgga tgtttgcgac atcccagaga gcttccagag aagtgttgat     660

ggcgtctctg aatccaagtt gcaaatctgt gttgaaccaa cttcccaaaa gctgatgcca     720

ggcagcacat tggttttaca gtgtgttgct gttggaagcc ctattcctca ctaccagtgg     780

ttcaaaaatg aattaccatt aacacatgag accaaaaagc tatacatggt gccttatgtg     840

gatttggaac accaaggaac ctactggtgt catgtatata atgatcgaga cagtcaagat     900

agcaagaagg tagaaatcat catagatgaa ttaaataatc ttggtcatcc tgataataaa     960

gagcaaacaa ctgaccagcc tttggcgaag gacaaggttg cccttttgat aggaaatatg    1020

aattaccggg agcaccccaa gctcaaagct cctttggtgg atgtgtacga attgactaac    1080

ttactgagac agctggactt caaagtggtt tcactgttgg atcttactga atatgagatg    1140

cgtaatgctg tggatgagtt tttactcctt ttagacaagg gagtatatgg gttattatat    1200

tatgcaggac atggttatga aaattttggg aacagcttca tggtccccgt tgatgctcca    1260

aatccatata ggtctgaaaa ttgtctgtgt gtacaaaata tactgaaatt gatgcaagaa    1320

aaagaaactg gacttaatgt gttcttattg gatatgtgta ggaaaagaaa tgactacgat    1380

gataccattc caatcttgga tgcactaaaa gtcaccgcca atattgtgtt tggatatgcc    1440

acgtgtcaag gagcagaagc ttttgaaatc cagcattctg gattggcaaa tggaatcttt    1500

atgaaatttt taaaagacag attattagaa gataagaaaa tcactgtgtt actggatgaa 1560

gttgcagaag atatgggtaa gtgtcacctt accaaaggca aacaggctct agagattcga 1620

agtagtttat ctgagaagag agcacttact gatccaatac agggaacaga atattctgct 1680

gaatctcttg tgcggaatct acagtgggcc aaggctcatg aacttccaga aagtatgtgt 1740

cttaagtttg actgtggtgt tcagattcaa ttaggatttg cagctgagtt ttccaatgtc 1800

atgatcatct atacaagtat agtttacaaa ccaccggaga taataatgtg tgatgcctac 1860

gttactgatt ttccacttga tctagatatt gatccaaaag atgcaaataa aggcacacct 1920

gaagaaactg gcagctactt ggtatcaaag gatcttccca agcattgcct ctataccaga 1980

ctcagttcac tgcaaaaatt aaaggaacat ctagtcttca cagtatgttt atcatatcag 2040

tactcaggat tggaagatac tgtagaggac aagcaggaag tgaatgttgg gaaacctctc 2100

attgctaaat tagacatgca tcgaggtttg ggaaggaaga cttgctttca aacttgtctt 2160

atgtctaatg gtccttacca gagttctgca gccacctcag gaggagcagg gcattatcac 2220

tcattgcaag acccattcca tggtgtttac cattcacatc ctggtaatcc aagtaatgtt 2280

acaccagcag atagctgtca ttgcagccgg actccagatg catttatttc aagtttcgct 2340

caccatgctt catgtcattt tagtagaagt aatgtgccag tagagacaac tgatgaaata 2400

ccatttagtt tctctgacag gctcagaatt tctgaaaaat ga 2442

<210> 2
<211> 2475
<212> DNA
<213> homo sapiens

<400> 2
atgtcgctgt tgggggaccc gctacaggcc ctgccgccct cggccgcccc cacggggccg 60

ctgctcgccc ctccggccgg cgcgaccctc aaccgcctgc gggagccgct gctgcggagg 120

ctcagcgagc tcctggatca ggcgcccgag ggccggggct ggaggagact ggcggagctg 180

gcggggagtc gcgggcgcct ccgcctcagt tgcctagacc tggagcagtg ttctcttaag 240

gtactggagc ctgaaggaag ccccagcctg tgtctgctga agttaatggg tgaaaaaggt 300

tgcacagtca cagaattgag tgatttcctg caggctatgg aacacactga agttcttcag 360

cttctcagcc ccccaggaat aaagattact gtaaacccag agtcaaaggc agtcttggct 420

ggacagtttg tgaaactgtg ttgccgggca actggacatc cttttgttca atatcagtgg 480

ttcaaaatga ataaagagat tccaaatgga aatacatcag agcttatttt taatgcagtg 540

catgtaaaag atgcaggctt ttatgtctgt cgagttaata acaatttcac ctttgaattc 600

agccagtggt cacagctgga tgtttgcgac atcccagaga gcttccagag aagtgttgat 660

ggcgtctctg aatccaagtt gcaaatctgt gttgaaccaa cttcccaaaa gctgatgcca 720

ggcagcacat tggttttaca gtgtgttgct gttggaagcc ctattcctca ctaccagtgg 780

ttcaaaaatg aattaccatt aacacatgag accaaaaagc tatacatggt gccttatgtg 840

gatttggaac accaaggaac ctactggtgt catgtatata atgatcgaga cagtcaagat 900

agcaagaagg tagaaatcat cataggaaga acagatgagg cagtggagtg cactgaagat 960

gaattaaata atcttggtca tcctgataat aaagagcaaa caactgacca gcctttggcg 1020

aaggacaagg ttgccctttt gataggaaat atgaattacc gggagcaccc caagctcaaa 1080

gctcctttgg tggatgtgta cgaattgact aacttactga gacagctgga cttcaaagtg 1140

gtttcactgt tggatcttac tgaatatgag atgcgtaatg ctgtggatga gtttttactc 1200

cttttagaca agggagtata tgggttatta tattatgcag gacatggtta tgaaaatttt 1260

gggaacagct tcatggtccc cgttgatgct ccaaatccat ataggtctga aaattgtctg 1320

tgtgtacaaa atatactgaa attgatgcaa gaaaaagaaa ctggacttaa tgtgttctta 1380

ttggatatgt gtaggaaaag aaatgactac gatgatacca ttccaatctt ggatgcacta 1440

aaagtcaccg ccaatattgt gtttggatat gccacgtgtc aaggagcaga agcttttgaa 1500

atccagcatt ctggattggc aaatggaatc tttatgaaat ttttaaaaga cagattatta 1560

gaagataaga aaatcactgt gttactggat gaagttgcag aagatatggg taagtgtcac 1620

cttaccaaag gcaaacaggc tctagagatt cgaagtagtt tatctgagaa gagagcactt 1680

actgatccaa tacagggaac agaatattct gctgaatctc ttgtgcggaa tctacagtgg 1740

gccaaggctc atgaacttcc agaaagtatg tgtcttaagt ttgactgtgg tgttcagatt 1800

caattaggat ttgcagctga gttttccaat gtcatgatca tctatacaag tatagtttac 1860

aaaccaccgg agataataat gtgtgatgcc tacgttactg attttccact tgatctagat 1920

attgatccaa aagatgcaaa taaaggcaca cctgaagaaa ctggcagcta cttggtatca 1980

aaggatcttc ccaagcattg cctctatacc agactcagtt cactgcaaaa attaaaggaa 2040

catctagtct tcacagtatg tttatcatat cagtactcag gattggaaga tactgtagag 2100

gacaagcagg aagtgaatgt tgggaaacct ctcattgcta aattagacat gcatcgaggt 2160

ttgggaagga agacttgctt tcaaacttgt cttatgtcta atggtcctta ccagagttct 2220

gcagccacct caggaggagc agggcattat cactcattgc aagacccatt ccatggtgtt 2280

taccattcac atcctggtaa tccaagtaat gttacaccag cagatagctg tcattgcagc 2340

cggactccag atgcatttat ttcaagtttc gctcaccatg cttcatgtca ttttagtaga 2400

agtaatgtgc cagtagagac aactgatgaa ataccattta gtttctctga caggctcaga 2460

atttctgaaa aatga 2475

<210> 3
<211> 813
<212> PRT
<213> homo sapiens

<400> 3

Met Ser Leu Leu Gly Asp Pro Leu Gln Ala Leu Pro Pro Ser Ala Ala
1               5                   10                  15

Pro Thr Gly Pro Leu Leu Ala Pro Pro Ala Gly Ala Thr Leu Asn Arg
            20                  25                  30

Leu Arg Glu Pro Leu Leu Arg Arg Leu Ser Glu Leu Leu Asp Gln Ala
        35                  40                  45

Pro Glu Gly Arg Gly Trp Arg Arg Leu Ala Glu Leu Ala Gly Ser Arg
    50                  55                  60

Gly Arg Leu Arg Leu Ser Cys Leu Asp Leu Glu Gln Cys Ser Leu Lys
65                  70                  75                  80

Val Leu Glu Pro Glu Gly Ser Pro Ser Leu Cys Leu Leu Lys Leu Met
                85                  90                  95

Gly Glu Lys Gly Cys Thr Val Thr Glu Leu Ser Asp Phe Leu Gln Ala
            100                 105                 110

Met Glu His Thr Glu Val Leu Gln Leu Leu Ser Pro Pro Gly Ile Lys
        115                 120                 125

Ile Thr Val Asn Pro Glu Ser Lys Ala Val Leu Ala Gly Gln Phe Val
    130                 135                 140

Lys Leu Cys Cys Arg Ala Thr Gly His Pro Phe Val Gln Tyr Gln Trp
145                 150                 155                 160

Phe Lys Met Asn Lys Glu Ile Pro Asn Gly Asn Thr Ser Glu Leu Ile
                165                 170                 175

Phe Asn Ala Val His Val Lys Asp Ala Gly Phe Tyr Val Cys Arg Val
            180                 185                 190

Asn Asn Asn Phe Thr Phe Glu Phe Ser Gln Trp Ser Gln Leu Asp Val
        195                 200                 205

Cys Asp Ile Pro Glu Ser Phe Gln Arg Ser Val Asp Gly Val Ser Glu
    210                 215                 220

Ser Lys Leu Gln Ile Cys Val Glu Pro Thr Ser Gln Lys Leu Met Pro
225                 230                 235                 240

Gly Ser Thr Leu Val Leu Gln Cys Val Ala Val Gly Ser Pro Ile Pro
                245                 250                 255

His Tyr Gln Trp Phe Lys Asn Glu Leu Pro Leu Thr His Glu Thr Lys
            260                 265                 270

Lys Leu Tyr Met Val Pro Tyr Val Asp Leu Glu His Gln Gly Thr Tyr
        275                 280                 285

Trp Cys His Val Tyr Asn Asp Arg Asp Ser Gln Asp Ser Lys Lys Val
    290                 295                 300

Glu Ile Ile Ile Asp Glu Leu Asn Asn Leu Gly His Pro Asp Asn Lys
305                 310                 315                 320

Glu Gln Thr Thr Asp Gln Pro Leu Ala Lys Asp Lys Val Ala Leu Leu
                325                 330                 335

Ile Gly Asn Met Asn Tyr Arg Glu His Pro Lys Leu Lys Ala Pro Leu
            340                 345                 350

Val Asp Val Tyr Glu Leu Thr Asn Leu Leu Arg Gln Leu Asp Phe Lys
        355                 360                 365

Val Val Ser Leu Leu Asp Leu Thr Glu Tyr Glu Met Arg Asn Ala Val
    370                 375                 380

Asp Glu Phe Leu Leu Leu Leu Asp Lys Gly Val Tyr Gly Leu Leu Tyr
385                 390                 395                 400

Tyr Ala Gly His Gly Tyr Glu Asn Phe Gly Asn Ser Phe Met Val Pro
                405                 410                 415

Val Asp Ala Pro Asn Pro Tyr Arg Ser Glu Asn Cys Leu Cys Val Gln
            420                 425                 430

Asn Ile Leu Lys Leu Met Gln Glu Lys Glu Thr Gly Leu Asn Val Phe
        435                 440                 445

Leu Leu Asp Met Cys Arg Lys Arg Asn Asp Tyr Asp Asp Thr Ile Pro
    450                 455                 460

Ile Leu Asp Ala Leu Lys Val Thr Ala Asn Ile Val Phe Gly Tyr Ala
465                 470                 475                 480

Thr Cys Gln Gly Ala Glu Ala Phe Glu Ile Gln His Ser Gly Leu Ala
                485                 490                 495

Asn Gly Ile Phe Met Lys Phe Leu Lys Asp Arg Leu Leu Glu Asp Lys
            500                 505                 510

Lys Ile Thr Val Leu Leu Asp Glu Val Ala Glu Asp Met Gly Lys Cys
        515                 520                 525

His Leu Thr Lys Gly Lys Gln Ala Leu Glu Ile Arg Ser Ser Leu Ser
    530                 535                 540

Glu Lys Arg Ala Leu Thr Asp Pro Ile Gln Gly Thr Glu Tyr Ser Ala
545                 550                 555                 560

Glu Ser Leu Val Arg Asn Leu Gln Trp Ala Lys Ala His Glu Leu Pro
                565                 570                 575

Glu Ser Met Cys Leu Lys Phe Asp Cys Gly Val Gln Ile Gln Leu Gly

580                 585                 590

Phe Ala Ala Glu Phe Ser Asn Val Met Ile Ile Tyr Thr Ser Ile Val
        595                 600                 605

Tyr Lys Pro Pro Glu Ile Ile Met Cys Asp Ala Tyr Val Thr Asp Phe
    610                 615                 620

Pro Leu Asp Leu Asp Ile Asp Pro Lys Asp Ala Asn Lys Gly Thr Pro
625                 630                 635                 640

Glu Glu Thr Gly Ser Tyr Leu Val Ser Lys Asp Leu Pro Lys His Cys
                645                 650                 655

Leu Tyr Thr Arg Leu Ser Ser Leu Gln Lys Leu Lys Glu His Leu Val
            660                 665                 670

Phe Thr Val Cys Leu Ser Tyr Gln Tyr Ser Gly Leu Glu Asp Thr Val
        675                 680                 685

Glu Asp Lys Gln Glu Val Asn Val Gly Lys Pro Leu Ile Ala Lys Leu
    690                 695                 700

Asp Met His Arg Gly Leu Gly Arg Lys Thr Cys Phe Gln Thr Cys Leu
705                 710                 715                 720

Met Ser Asn Gly Pro Tyr Gln Ser Ser Ala Ala Thr Ser Gly Gly Ala
                725                 730                 735

Gly His Tyr His Ser Leu Gln Asp Pro Phe His Gly Val Tyr His Ser
            740                 745                 750

His Pro Gly Asn Pro Ser Asn Val Thr Pro Ala Asp Ser Cys His Cys
        755                 760                 765

Ser Arg Thr Pro Asp Ala Phe Ile Ser Ser Phe Ala His His Ala Ser
    770                 775                 780

Cys His Phe Ser Arg Ser Asn Val Pro Val Glu Thr Thr Asp Glu Ile
785                 790                 795                 800

Pro Phe Ser Phe Ser Asp Arg Leu Arg Ile Ser Glu Lys
                805                 810

<210>  4
<211>  824
<212>  PRT
<213>  homo sapiens

<400>  4

Met Ser Leu Leu Gly Asp Pro Leu Gln Ala Leu Pro Pro Ser Ala Ala
1               5                   10                  15

Pro Thr Gly Pro Leu Leu Ala Pro Pro Ala Gly Ala Thr Leu Asn Arg
            20                  25                  30

Leu Arg Glu Pro Leu Leu Arg Arg Leu Ser Glu Leu Leu Asp Gln Ala
        35                  40                  45

Pro Glu Gly Arg Gly Trp Arg Arg Leu Ala Glu Leu Ala Gly Ser Arg
    50                  55                  60

Gly Arg Leu Arg Leu Ser Cys Leu Asp Leu Glu Gln Cys Ser Leu Lys
65                  70                  75                  80

Val Leu Glu Pro Glu Gly Ser Pro Ser Leu Cys Leu Leu Lys Leu Met
                85                  90                  95

Gly Glu Lys Gly Cys Thr Val Thr Glu Leu Ser Asp Phe Leu Gln Ala
            100                 105                 110

Met Glu His Thr Glu Val Leu Gln Leu Leu Ser Pro Pro Gly Ile Lys
        115                 120                 125

Ile Thr Val Asn Pro Glu Ser Lys Ala Val Leu Ala Gly Gln Phe Val
    130                 135                 140

Lys Leu Cys Cys Arg Ala Thr Gly His Pro Phe Val Gln Tyr Gln Trp
145                 150                 155                 160

Phe Lys Met Asn Lys Glu Ile Pro Asn Gly Asn Thr Ser Glu Leu Ile
                165                 170                 175

Phe Asn Ala Val His Val Lys Asp Ala Gly Phe Tyr Val Cys Arg Val
            180                 185                 190

Asn Asn Asn Phe Thr Phe Glu Phe Ser Gln Trp Ser Gln Leu Asp Val
        195                 200                 205

Cys Asp Ile Pro Glu Ser Phe Gln Arg Ser Val Asp Gly Val Ser Glu
    210                 215                 220

Ser Lys Leu Gln Ile Cys Val Glu Pro Thr Ser Gln Lys Leu Met Pro
225                 230                 235                 240

Gly Ser Thr Leu Val Leu Gln Cys Val Ala Val Gly Ser Pro Ile Pro
                245                 250                 255

His Tyr Gln Trp Phe Lys Asn Glu Leu Pro Leu Thr His Glu Thr Lys
            260                 265                 270

Lys Leu Tyr Met Val Pro Tyr Val Asp Leu Glu His Gln Gly Thr Tyr
        275                 280                 285

Trp Cys His Val Tyr Asn Asp Arg Asp Ser Gln Asp Ser Lys Lys Val
    290                 295                 300

Glu Ile Ile Ile Gly Arg Thr Asp Glu Ala Val Glu Cys Thr Glu Asp
305                 310                 315                 320

Glu Leu Asn Asn Leu Gly His Pro Asp Asn Lys Glu Gln Thr Thr Asp
                325                 330                 335

Gln Pro Leu Ala Lys Asp Lys Val Ala Leu Leu Ile Gly Asn Met Asn
            340                 345                 350

Tyr Arg Glu His Pro Lys Leu Lys Ala Pro Leu Val Asp Val Tyr Glu
        355                 360                 365

Leu Thr Asn Leu Leu Arg Gln Leu Asp Phe Lys Val Val Ser Leu Leu
    370                 375                 380

Asp Leu Thr Glu Tyr Glu Met Arg Asn Ala Val Asp Glu Phe Leu Leu
385                 390                 395                 400

Leu Leu Asp Lys Gly Val Tyr Gly Leu Leu Tyr Tyr Ala Gly His Gly
                405                 410                 415

Tyr Glu Asn Phe Gly Asn Ser Phe Met Val Pro Val Asp Ala Pro Asn
            420                 425                 430

Pro Tyr Arg Ser Glu Asn Cys Leu Cys Val Gln Asn Ile Leu Lys Leu
        435                 440                 445

Met Gln Glu Lys Glu Thr Gly Leu Asn Val Phe Leu Leu Asp Met Cys
    450                 455                 460

Arg Lys Arg Asn Asp Tyr Asp Asp Thr Ile Pro Ile Leu Asp Ala Leu
465                 470                 475                 480

Lys Val Thr Ala Asn Ile Val Phe Gly Tyr Ala Thr Cys Gln Gly Ala
                485                 490                 495

Glu Ala Phe Glu Ile Gln His Ser Gly Leu Ala Asn Gly Ile Phe Met
            500                 505                 510

Lys Phe Leu Lys Asp Arg Leu Leu Glu Asp Lys Lys Ile Thr Val Leu
        515                 520                 525

Leu Asp Glu Val Ala Glu Asp Met Gly Lys Cys His Leu Thr Lys Gly
    530                 535                 540

Lys Gln Ala Leu Glu Ile Arg Ser Ser Leu Ser Glu Lys Arg Ala Leu
545                 550                 555                 560

Thr Asp Pro Ile Gln Gly Thr Glu Tyr Ser Ala Glu Ser Leu Val Arg

                565                 570                 575

Asn Leu Gln Trp Ala Lys Ala His Glu Leu Pro Glu Ser Met Cys Leu
            580                 585                 590

Lys Phe Asp Cys Gly Val Gln Ile Gln Leu Gly Phe Ala Ala Glu Phe
        595                 600                 605

Ser Asn Val Met Ile Ile Tyr Thr Ser Ile Val Tyr Lys Pro Pro Glu
    610                 615                 620

Ile Ile Met Cys Asp Ala Tyr Val Thr Asp Phe Pro Leu Asp Leu Asp
625                 630                 635                 640

Ile Asp Pro Lys Asp Ala Asn Lys Gly Thr Pro Glu Glu Thr Gly Ser
                645                 650                 655

Tyr Leu Val Ser Lys Asp Leu Pro Lys His Cys Leu Tyr Thr Arg Leu
            660                 665                 670

Ser Ser Leu Gln Lys Leu Lys Glu His Leu Val Phe Thr Val Cys Leu
        675                 680                 685

Ser Tyr Gln Tyr Ser Gly Leu Glu Asp Thr Val Glu Asp Lys Gln Glu
    690                 695                 700

Val Asn Val Gly Lys Pro Leu Ile Ala Lys Leu Asp Met His Arg Gly
705                 710                 715                 720

Leu Gly Arg Lys Thr Cys Phe Gln Thr Cys Leu Met Ser Asn Gly Pro
                725                 730                 735

Tyr Gln Ser Ser Ala Ala Thr Ser Gly Gly Ala Gly His Tyr His Ser
            740                 745                 750

Leu Gln Asp Pro Phe His Gly Val Tyr His Ser His Pro Gly Asn Pro
        755                 760                 765

Ser Asn Val Thr Pro Ala Asp Ser Cys His Cys Ser Arg Thr Pro Asp
    770                 775                 780

Ala Phe Ile Ser Ser Phe Ala His His Ala Ser Cys His Phe Ser Arg
785                 790                 795                 800

Ser Asn Val Pro Val Glu Thr Thr Asp Glu Ile Pro Phe Ser Phe Ser
                805                 810                 815

Asp Arg Leu Arg Ile Ser Glu Lys
            820

<210> 5
<211> 19

<212>  RNA
<213>  artificial

<220>
<223>  siGenome Smart pool siRNA D-005936-02 Malt1

<400>  5
ggaaaccucu cauugcuaa                                                    19


<210>  6
<211>  19
<212>  RNA
<213>  artificial

<220>
<223>  siGenome Smart pool siRNA D-005936-03 Malt1

<400>  6
agagacaacu gaugaaaua                                                    19


<210>  7
<211>  19
<212>  RNA
<213>  artificial

<220>
<223>  siGenome Smart pool siRNA D-005936-05 Malt1

<400>  7
gggaguauau ggguuauua                                                    19


<210>  8
<211>  19
<212>  RNA
<213>  artificial

<220>
<223>  siGenome Smart pool siRNA D-005936-018 Malt1

<400>  8
guauauaaug aucgagaca                                                    19


<210>  9
<211>  20
<212>  DNA
<213>  artificial

<220>
<223>  primer

<400>  9
tttgagtctg cccagttccc                                                   20


<210>  10
<211>  20
<212>  DNA
<213>  artificial

<220>
<223>  primer

<400>  10
tcagttatat cctggccgcc                                                   20

<210>  11
<211>  19
<212>  DNA
<213>  artificial

<220>
<223>  primer

<400>  11
gcttgcaaag gatccacca                                                    19


<210>  12
<211>  21
<212>  DNA
<213>  artificial

<220>
<223>  primer

<400>  12
tccgatccta gcagcttctc a                                                 21


<210>  13
<211>  20
<212>  DNA
<213>  artificial

<220>
<223>  primer

<400>  13
tgcaataacc acccctgacc                                                   20


<210>  14
<211>  21
<212>  DNA
<213>  artificial

<220>
<223>  primer

<400>  14
tgcgcagaat gagatgagtt g                                                 21


<210>  15
<211>  20
<212>  DNA
<213>  artificial

<220>
<223>  primer

<400>  15
ctccagaagg ccagacaaac                                                   20


<210>  16
<211>  21
<212>  DNA
<213>  artificial

<220>
<223>  primer

<400>  16
aatggtaaac aggcctccac t                                                21


<210>  17
<211>  22
<212>  DNA
<213>  artificial

<220>
<223>  primer

<400>  17
ccagagcagt gaggtcttag gc                                               22


<210>  18
<211>  18
<212>  DNA
<213>  artificial

<220>
<223>  primer

<400>  18
tgtgaagcag caggagcg                                                    18


<210>  19
<211>  22
<212>  DNA
<213>  artificial

<220>
<223>  primer

<400>  19
catgtcggac tacgagaacg at                                               22


<210>  20
<211>  21
<212>  DNA
<213>  artificial

<220>
<223>  primer

<400>  20
caggtaaggt gtgatgcgtg a                                                21


<210>  21
<211>  21
<212>  DNA
<213>  artificial

<220>
<223>  primer

<400>  21
atggagccac gaacaacctc t                                                21


<210>  22
<211>  21
<212>  DNA
<213>  artificial

<220>
<223>  primer

<400>  22
tcgtgctgga ttctccttct g                                                21


<210>  23
<211>  22
<212>  DNA
<213>  artificial

<220>
<223>  primer

<400>  23
gacccattcc atggtgttta cc                                               22


<210>  24
<211>  22
<212>  DNA
<213>  artificial

<220>
<223>  primer

<400>  24
aataaatgca tctggagtcc gg                                               22


<210>  25
<211>  20
<212>  DNA
<213>  artificial

<220>
<223>  primer

<400>  25
ccagagacaa caacggctcc                                                  20


<210>  26
<211>  20
<212>  DNA
<213>  artificial

<220>
<223>  primer

<400>  26
tgtcgatgcg atagtgcagc                                                  20


<210>  27
<211>  20
<212>  DNA
<213>  artificial

<220>
<223>  primer

<400>  27
ctggccaaca tggtgaaacc                                                  20


<210>  28
<211>  19

<212>  DNA
<213>  artificial

<220>
<223>  primer

<400>  28
caggcacgta ccaccatgc                                                    19


<210>  29
<211>  20
<212>  DNA
<213>  artificial

<220>
<223>  primer

<400>  29
ccatgttcgt catgggtgtg                                                   20


<210>  30
<211>  21
<212>  DNA
<213>  artificial

<220>
<223>  primer

<400>  30
ggtgctaagc agttggtggt g                                                 21


<210>  31
<211>  23
<212>  DNA
<213>  artificial

<220>
<223>  primer

<400>  31
ctgtgtgtct tccactttgt ccc                                               23


<210>  32
<211>  22
<212>  DNA
<213>  artificial

<220>
<223>  primer

<400>  32
tgcattgttt tcctgacaat cg                                                22


<210>  33
<211>  18
<212>  DNA
<213>  artificial

<220>
<223>  primer

<400>  33
catcccaggc ctctagcc                                                     18

<210>  34
<211>  22
<212>  DNA
<213>  artificial

<220>
<223>  primer

<400>  34
ggtttggttc cctcagttgt tc                                                22


<210>  35
<211>  22
<212>  DNA
<213>  artificial

<220>
<223>  primer

<400>  35
acgacctaag ctgcactttt cc                                                22


<210>  36
<211>  22
<212>  DNA
<213>  artificial

<220>
<223>  primer

<400>  36
gagcctcaga catctccagt cc                                                22


<210>  37
<211>  19
<212>  DNA
<213>  artificial

<220>
<223>  primer

<400>  37
gagtactcag cccgccagg                                                    19


<210>  38
<211>  19
<212>  DNA
<213>  artificial

<220>
<223>  primer

<400>  38
cctctttgca ggagacggc                                                    19


<210>  39
<211>  21
<212>  DNA
<213>  artificial

<220>
<223>  primer

<400>  39
tcttgaaatt cccccagaag g                                                21


<210>  40
<211>  19
<212>  DNA
<213>  artificial

<220>
<223>  primer

<400>  40
ggacggagag tccaatggc                                                   19


<210>  41
<211>  20
<212>  DNA
<213>  artificial

<220>
<223>  primer

<400>  41
tactagcggt tttacgggcg                                                  20


<210>  42
<211>  24
<212>  DNA
<213>  artificial

<220>
<223>  primer

<400>  42
tcgaacagga ggagcagaga gcga                                             24